# EUROPEAN PATENT APPLICATION

(11) **EP 3 831 926 A1**
(43) Date of publication of application: **09.06.2021**
(21) Application number: 19848553.4
(22) Date of filing: 06.08.2019
(51) Int. Cl.: C12M 3/08, C12M 3/00

(54) **CELL CULTURE SYSTEM AND CELL MASS PRODUCTION METHOD USING SAME**

(30) Priority: 06.08.2018 JP 2018148042; 11.09.2018 JP 2018170103
(71) Applicant: Nissan Chemical Corporation, Tokyo 103-6119 (JP)
(72) Inventor: IWAKAMI, Masashi, Shiraoka-shi, Saitama 349-0294 (JP); OTSUKA, Keiichiro, Shiraoka-shi, Saitama 349-0294 (JP); ANNO, Shiho, Shiraoka-shi, Saitama 349-0294 (JP); HATANAKA, Daisuke, Funabashi-shi, Chiba 274-0052 (JP); HAYASHI, Hisato, Tokyo 103-6119 (JP)
(74) Representative: J A Kemp LLP
(86) International application number: PCT/JP2019/030939
(87) International publication number: WO 2020/032041

(57) **Abstract**

A cell culture system for dividing and subculturing cell aggregates is constituted by connecting a first container 10 and a divider 20. An outlet of a divider is connected to the first container. A mesh structure 22 for dividing a cell aggregate is provided in a conduit for dividing in the divider 20, and a cell aggregate is divided while passing through the mesh structure. The divided cell aggregate is sent to the first container 10 without contacting the outside air in a preferred embodiment, where further culture is performed. This makes it possible to divide the cell aggregate and perform suspension culture while maintaining the closed system. The division and suspension culture of cell aggregates can be repeated by sending the cultured cell aggregates to a divider of its own cell culture system or a divider of a cell culture system prepared separately.

## Description

### [Technical Field]

The present invention relates to a cell culture system that makes it possible to repeat division of cell aggregates obtained by growing cells in suspension culture, and further suspension culture thereof, in a closed system, and a method for producing cell aggregates by using the cell culture system.

### [Background Art]

In recent years, a cell culture method (also called a suspension culture method) has been developed in which various cells such as pluripotent stem cells and the like are suspended in a liquid medium and three-dimensionally grown into a cell aggregate (e.g., patent document 1 and the like). In addition, a liquid medium for preferably performing the suspension culture method and a production method thereof have also been developed (e.g., patent document 1 and the like).

In suspension culture method, the undifferentiated state of pluripotent stem cells may decrease as the cell aggregate grows larger. For example, non-patent document 1 suggests that the undifferentiated state of large cell aggregates of 150 µm or more may decrease.

On the other hand, in the culture method of pluripotent stem cells described in patent document 1, pluripotent stem cells are suspension cultured until they become large cell aggregates having an average diameter of about 200 - about 300 µm, the obtained large cell aggregates are divided into smaller cell aggregates having an average diameter of about 80 to about 120 µm, after which suspension culture is further continued to maintain and amplify the pluripotent stem cells.

### [Document List]

### [Patent documents]

patent document 1: WO 2013/077423
patent document 2: WO 2016/163444

### [non-patent document]

non-patent document 1: Andreas Elanzew et al., "A reproducible and versatile system for the dynamic expansion of human pluripotent stem cells in suspension", Biotechnology Journal, 2015, 10, 1589-1599.

### [Summary of Invention]

### [Technical Problem]

The present inventors studied in detail the culture method of a pluripotent stem cell described in patent document 1 and found that the method includes the following problem to be improved.

In the aforementioned culture method, the step of dividing the cell aggregate and the step of further culturing the divided cell aggregate are performed using a container and a dividing instrument separate from each other. Therefore, the cell aggregate and a liquid medium containing same come into contact with the outside air when they are sent to the division step, and the cell aggregate divided in the division step and the liquid medium containing same come into contact with the outside air when they are sent to a culture container in the next stage for further culture. Therefore, when the step of dividing the cell aggregate and the step of further culturing the divided cell aggregate are repeated, the cell aggregate and the liquid medium come into contact with the outside air many times and may be contaminated.

The present invention aims to provide a cell culture system that can solve the above-mentioned problems, and make it possible to perform division of cell aggregates and further suspension culture of the divided cells in a closed system, and a method for producing cell aggregates by using the cell culture system.

### [Solution to Problem]

The main constitution of the present invention is as follows.
[1] A cell culture system for dividing and subculturing a cell aggregate, comprising at least:
   a divider for dividing a cell aggregate to be divided which is supplied together with a liquid medium from a supply source into smaller cell aggregates; and
   a first container for suspension culturing the cell aggregates divided by the divider;
      wherein,
   the divider has an inlet, an internal dividing conduit, and an outlet, the inlet is constituted such that the cell aggregate to be divided and the liquid medium are received from the supply source into the dividing conduit, the dividing conduit is provided with a mesh structure to divide the cell aggregate to be divided, the cell aggregate to be divided is divided when passing through the mesh structure together with the liquid medium, and the outlet is connected to the first container so as to deliver the divided cell aggregates to the first container, and
   the first container has a constitution for receiving the divided cell aggregates and the liquid medium and sending out the cell aggregates suspension cultured in the first container.
[2] The cell culture system according to [1], wherein the first container is a flexible cell culture bag.
[3] The cell culture system according to [1] or [2], wherein the mesh structure is a mesh woven with wire.
[4] The cell culture system according to [1] or [2], wherein the mesh structure is a porous film with many through-holes disposed on the film surface, the mesh structure comprises many through-holes penetrating the predetermined region in the film thickness direction, and a beam part serving as a partition between the through-holes,
   the through-holes have an opening shape of a size permitting passage of the aforementioned smaller cell aggregates,
   and the beam part is a remainder after subtracting the through-hole from the main body part in the predetermined region, is a part that cuts the cell aggregates to be divided, and is integrally connected to form a network.
[5] The cell culture system according to [4], wherein
   a cross-sectional shape in the perpendicular longitudinal direction of the aforementioned beam part is a rectangle, or two corners on the inlet side of the rectangle have a round shape.
[6] The cell culture system according to [4] or [5], wherein
   said many through-holes have opening shapes of quadrangles congruent with each other, and said beam parts are connected to each other in an orthogonal lattice pattern, or said many through-holes have opening shapes of hexagons congruent with each other, and said beam parts are connected to each other in a honeycomb-shape.
[7] The cell culture system according to any of [1] to [6], further comprising a collector for recovering cell aggregates with a predetermined size, wherein
   the collector is connected to the first container so as to receive the cell aggregates suspension cultured in the first container and the liquid medium,
   the collector has a separation chamber, the separation chamber is divided into a first chamber and a second chamber by a mesh structure for separation, and an internal flow path is configured such that the liquid medium that has entered the collector moves from the first chamber through the mesh structure for separation to the second chamber and exits the collector, and
   the mesh structure for separation has mesh-holes of a predetermined size to prevent cell aggregates of a size intended for collection from passing through, whereby, of the cell aggregates that entered the collector together with the liquid medium, a cell aggregate of a size intended for collection does not pass through the mesh structure for separation and stays in the first chamber.
[8] The cell culture system according to [7], wherein an overall shape of the mesh structure for separation is sheet-like or sac-like.
[9] The cell culture system according to [7] or [8], wherein the mesh structure for separation is configured in a separation chamber of the collector such that the liquid medium has a vertically upward directional component in the progress direction when the liquid medium passes through mesh-holes of the mesh structure for separation in the collector.
[10] The cell culture system according to any of [7] to [9], wherein
   the first chamber in the separation chamber of the collector comprises
   a first inlet port for receiving the cell aggregates suspension cultured in the first container and the liquid medium in the first chamber,
   a second inlet port for flowing a liquid for diluting a reagent and/or the suspension containing the cell aggregates and the liquid medium into the first chamber from the outside, and
   an outlet port for flowing a part of the cell aggregates from the first chamber to the outside.
[11] The cell culture system according to any of [1] to [10], further comprising a liquid feeding device for moving the liquid medium and the cell aggregates, wherein the liquid feeding device is one or more selected from the group consisting of an apparatus for deforming the first container by compression to send out the liquid medium and the cell aggregates contained therein, a syringe pump, and a peristaltic pump.
[12] The cell culture system according to any of [1] to [11], further comprising
   a backward direction liquid feeding function or a backward direction liquid feeding device for moving the predetermined liquid such that the predetermined liquid passes through the mesh structure in a direction opposite to the direction of passage of the cell aggregate to be divided through the mesh structure together with the liquid medium.
[13] The cell culture system according to any of [1] to [12], wherein
   the cell aggregate to be divided is a cell aggregate composed of pluripotent stem cells.
[14] The cell culture system according to any of [1] to [13], wherein
   the liquid medium comprises a fine structure for suspending the cells or cell aggregates in the liquid medium, and the fine structure is dispersed and floating in the liquid medium.
[15] A method for producing a cell aggregate by using the cell culture system of any of the above-mentioned [1] to [14], comprising
   a step of dividing a cell aggregate to be divided and a liquid medium by passing the cell aggregate through the divider in the cell culture system, and
   a step of feeding the cell aggregate divided in the divider into the first container together with a liquid medium, and performing suspension culture in the first container.
[16] The production method according to [15], wherein
   the flow velocity of the liquid medium is 10 mm/sec - 500 mm/sec when the cell aggregate passes through the aforementioned divider together with the liquid medium.
[17] The method according to [15] or [16], further comprising a backflow washing step, wherein the backflow washing step is a step of passing, after division of a predetermined amount of the cell aggregates in the aforementioned step of dividing the cell aggregates, a predetermined liquid through the mesh structure in the divider direction opposite to the direction of passage of the cell aggregate through the mesh structure of the device for division, thereby washing the mesh structure.
[18] The production method according to any of the above-mentioned [15] to [17],
   wherein
   the cell culture system is the cell culture system of any of the above-mentioned [7] to [10],
   the production method further comprising a step of feeding the cell aggregates suspension cultured in the first container into the collector of the cell culture system together with the liquid medium, and feeding and collecting cell aggregates remaining in the first chamber of the collector in a collection container.
[19] The production method according to [17], wherein a flow velocity of the liquid medium passing through the collector for separation of the cell aggregates from the liquid medium is 0.01 mm/sec - 25 mm/sec.

### [Advantageous Effects of Invention]

The cell culture system and production method according to the present invention allow the cell aggregate to move in the closed system together with the liquid medium. In this movement within the closed system, it is also possible to perform division of the cell aggregate and growth of the cell aggregate after the division. The liquid medium is provided with a port so that a new liquid medium may be appropriately injected and an old liquid medium may be appropriately removed from the port. Therefore, it is possible to automatically proliferate a large amount of the target cell aggregates while suppressing contamination from the outside.

In addition, the cell aggregate cultured in the closed system in a preferable embodiment described above is transferred to a collector together with a liquid medium (preferably, transferred to a collector without exposure to the outside air), and the cell aggregate is safely collected. A part or all of the collected cell aggregate may be returned to the cell culture system again, or transferred to other cell culture system prepared separately in the required number, and the division and culture of the cell aggregate may be repeated as many times as necessary. Such other cell culture systems prepared in the required number may be arranged in parallel so that the cell aggregate will be distributed from one system to a plurality of systems, or arranged in series so that they may be sequentially supplied from one system to the next system.

### [Brief Description of Drawings]

Fig. 1 schematically shows one embodiment of the constitution of the cell culture system according to the present invention. In the Figure, for the sake of explanation, the culture container is drawn small and the pipe line is drawn thick, but in reality, the culture container is of the size of a general culture bag, and the pipe line is a flexible piping tube. The mesh structure of the divider is drawn with a thick dotted line.
Fig. 2 shows the structure of a mesh used for dividing cell aggregates. Fig. 2(a) is a partially enlarged view showing a predetermined region of the sheet surface of the mesh, and Fig. 2(b) is a cross-sectional view taken along the line X1-X1 of Fig. 2(a). In Fig. 2(b), the cross section of the wire is hatched.
Fig. 3 shows one example of an embodiment of the porous film proposed as the mesh structure of a preferable divider. Fig. 3(a) is a partially enlarged view of a predetermined region of the film surface of the film-like main body part, and Fig. 3(b) is a cross-sectional view taken along the line X2-X2 of Fig. 3(a). Figs. 3(c) - (e) show other embodiment of the cross section of the beam part shown in Fig. 3(b). In Figs. 3(b) - (e), the cross section of the beam part is hatched.
Fig. 4 illustrates the region where the mesh structure is provided on the film surface in the porous film proposed in the present invention. In this figure, the opening of the through-hole is not shown, and the area where the mesh structure is provided is shown by hatching.
Fig. 5 shows other example of the porous film proposed in the present invention, and is a partially enlarged view of a predetermined region of the film surface of the main body part.
Fig. 6 is a cross-sectional view showing one embodiment of the structure of a holder configured so that the mesh structure can be preferably used as a divider.
Fig. 7 schematically shows one embodiment of the constitution of the cell culture system added with a collector according to the present invention. The mesh structure for separation in the collector is drawn with a thick dotted line. In the Figure, for the sake of explanation, the culture container is drawn small and the pipe line is drawn thick, as in Fig. 1. The flow of the cell culture is shown with thick arrows, and a flow relating to collection using the collector is shown with arrows of thin broken line.
Fig. 8 shows one embodiment of a preferred embodiment of the collector. Fig. 8(a) shows the inside of the collector by removing the side wall, and Fig. 8(b) is a sectional view of Fig. 8(a) along X3-X3.
Fig. 9 shows another embodiment of a preferred embodiment of the collector. Fig. 9(a) shows the inside of the collector by removing the side wall, and Fig. 9(b) is a sectional view of Fig. 9(a) along X4-X4.
Fig. 10 is a sectional view showing other embodiment of a preferred embodiment of the collector. In the Figure, the section of the mesh structure for separation is shown with a thick dotted line. The interval between the dots in the dotted line and the actual size of the mesh-holes are not related. The cell aggregates are shown with white circular symbols. The same applies to Fig. 11 and Fig. 15.
Fig. 11 is a sectional view showing other embodiment of a preferred embodiment of the collector.
Fig. 12 is a sectional view schematically showing another embodiment of a preferred embodiment of the collector.
Fig. 13 shows another embodiment of a preferred embodiment of the collector. Fig. 13(a) shows the inside of the collector by removing the side wall, and Fig. 13(b) is a sectional view of Fig. 13(a) along X5-X5.
Fig. 14 shows another embodiment of a preferred embodiment of the collector. Fig. 14(a) shows the inside of the collector by removing the side wall. Fig. 14(b) is a perspective view schematically showing one embodiment of the constitution of the collector of Fig. 14(a). Fig. 14(c) is a perspective view schematically showing other embodiment of the constitution of the collector of Fig. 14(a).
Fig. 15 is a sectional view showing other embodiment of a preferred embodiment of the collector.
Fig. 16 is a flowchart showing one embodiment of a cell culture method using the method for dividing a cell aggregate according to the present invention.
Fig. 17 is a graph showing the test results of examining the effect of backflow washing on the device according to the present invention. Fig. 17(a), (b) are graphs showing the survival rate of cells contained in the divided cell aggregate when the cell aggregate division is continued without performing backflow washing. Fig. 17(c), (d) are graphs showing the survival rate of cells contained in the divided cell aggregate when the cell aggregate division is continued while performing backflow washing when a predetermined amount is divided.
Fig. 18 is a graph showing the test results of investigation of the relationship between the cross-sectional shape of the beam part of a mesh structure, and the division performance in the present invention.
Fig. 19 is a block diagram showing the constitution of the cell culture system used in the Examples of the present invention.

### [Description of Embodiments]

Hereinafter, the constitution of the cell culture system of the present invention (also referred to as the cell culture system) is described in detail with reference to examples.

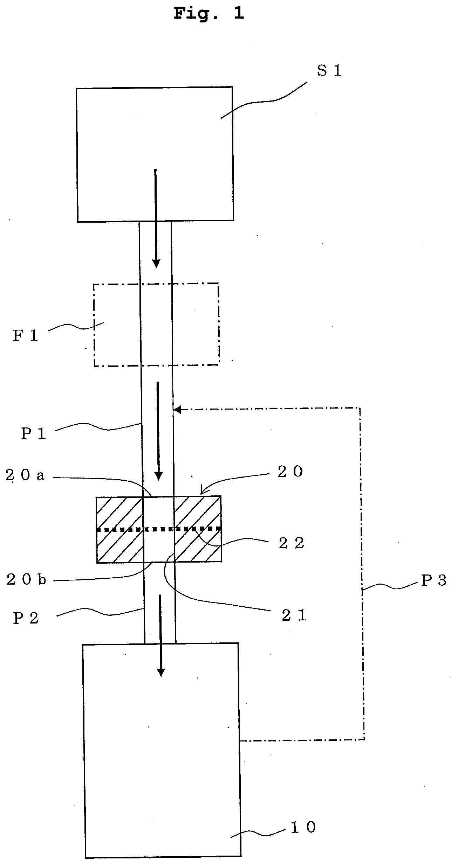

In a preferred embodiment, the cell culture system is configured to be an entirely closed system (a system that is shielded from the outside air except for the permeation of gas molecules), the cell aggregate is divided in a conduit isolated from the outside, and the cell aggregate after division is further cultured (subculture). The cultured cell aggregate may then be fed back into the same cell culture system and then sent to another cell culture system where further division and culture may be repeated. As shown in Fig. 1 as an example of a preferred embodiment, the cell culture system comprises at least a divider 20 for dividing the cell aggregate to be divided and supplied with the liquid medium from the source S1, and a first container 10 for suspension culturing the cell aggregate divided by the divider 20, and these are air-tightly connected so that the division and culture of the cell aggregate in such closed system can be repeated. In the example of Fig. 1, while the divider 20 and the first container 10 are connected via the piping P2, they may be directly connected to each other. F1 is a liquid feeding device (pump) for sending a liquid medium and a cell aggregate along a conduit. The position of the liquid feeding device F1 is not limited. The liquid feeding device is described later.

### (Supply source)

The supply source S1 shown in Fig. 1 supplies the cell aggregate to be divided and the liquid medium (i.e., the liquid medium and the cell aggregate present in the solution thereof) to the cell culture system. The supply source S1 may be an external culture container containing the cell aggregate and the liquid medium, or a collection container attached to the collector provided in the cell culture system. In the example of Fig. 1, a cell aggregate obtained by detaching colonies grown by adhesion culture from the scaffold (or cell aggregate formed by suspension culture) in the external cell culture apparatus is placed in a container, such as syringe and the like, together with a liquid medium, and fed into the divider 20 through the conduit P1 without contacting the outside air in a preferred embodiment. At that time, a liquid medium in an amount required for culturing after division may be added from a confluence conduit or the like, or a liquid medium and cell aggregate in the amounts required for culturing after division may be stored in the supply source S1. Piping for replacement of a liquid medium required for culturing and a liquid medium to be discarded may be added as appropriate.

When the collector attached to the cell culture system is the supply source, the collector may be provided on the conduit P3, and division and culture of the cell aggregate may be preferably repeated in circulation in the closed loop. An open-close valve, switching valve, container, liquid feeding device and the like may be appropriately provided so that the division and culture of the cell aggregate are preferably repeated in circulation in the closed loop.

A constitution in which each cell culture system becomes a supply source for the cell culture system in the next stage may be configured, where a plurality of the cell culture systems is used, the collection container of the cell culture system in the first stage is used as the supply source S1, the cell aggregate and the liquid medium supplied therefrom are supplied to the cell culture system in the second and subsequent stages.

### (Divider)

As shown in Fig. 1, the divider 20 has an inlet 20a and an outlet 20b, the outlet 20b being air-tightly connected to the first container 10 (i.e., preferably preventing outside air from entering). In the example of Fig. 1, the outlet of the divider 20 and the inlet of the first container 10 are connected via a conduit P2 (a piping member such as a soft resin tube and the like), but the both may be directly connected. In addition, a switching valve, an injection port, or the like may be connected in the middle of the conduit P2 as necessary.

The divider 20 has a dividing conduit 21 that connects the inlet 20a and the outlet 20b. A mesh structure 22 for dividing (crushing) a cell aggregate is arranged in the dividing conduit 21, and it is configured that the cell aggregate sent out from the first container 10 together with the liquid medium into the dividing conduit 21 passes through the mesh structure 22 without contacting the outside air in a preferred embodiment. As used herein, "pass through the mesh structure" means passing through the mesh-holes of the mesh structure from one side to the other.

Preferred embodiments of the mesh structure and a structure that preferably holds the mesh structure are described later.

### (The first container)

The first container 10 has a constitution capable of containing the liquid medium and the divided cell aggregate without contact with the outside air in a preferred embodiment, and a constitution capable of sending out the contained liquid medium and the cell aggregate (cell aggregates dispersed in the liquid medium) without contact with the outside air in a preferred embodiment.

In the preferred embodiment of the present invention, (liquid medium and cell aggregate are free of contact with the outside air) means that the liquid medium and/or cell aggregate do(es) not come into direct contact with or are(is) not exposed to the outside air. For example, when the outside air (oxygen, etc.) permeates into the container through a gas permeable film, it does not correspond to the contact. When a specific gas is actively injected into the container for cell culture, the gas injected with the intention of contributing to such culture is not the outside air, and therefore, does not correspond to the contact with the outside air.

Examples of the aforementioned first container 10 include a flexible cell culture bag, a flask, and the like, and a container having a variable volume is preferable. In particular, a cell culture bag using a flexible polymer film for the wall is inexpensive, has a large volume, and contracts and expands as the liquid medium and cell aggregate enter and exit (it is not necessary to supplementally enter/exit the outside air according to the entry/exit of the liquid medium and the cell aggregate), and is a preferable container in which the liquid medium and the cell aggregate do not come into contact with the outside air. As the cell culture bag, conventionally-known ones such as gas permeable one and the like can be used, and conventionally-known flasks can also be used. These having a volume as required can be selected.

### (Mesh structure of divider)

As described above, the divider has a dividing conduit, and a mesh structure for dividing the cell aggregate is arranged in the dividing conduit. As the cell aggregate passes through this mesh structure, it is divided into sizes according to the size of the mesh-hole. Whether cell aggregates larger than the mesh-hole of the mesh structure are divided by the mesh structure and pass through the mesh structure, or they cannot pass through the mesh structure and remain on the sheet surface of the mesh structure is mainly determined by the flow velocity of the liquid medium that passes through the mesh structure and the thickness of the wires and the beam part (described later) constituting the mesh structure.

The mesh structure may be a mesh woven with wire, or may be a porous film in which a large number of through-holes are arranged in a predetermined region on the main surface of the film. The division of a cell aggregate by a mesh woven with wire itself is described in the above-mentioned patent document 1. Therefore, known meshes used for such cutting can be used. The present invention proposes a holder described later for incorporating a mesh or a porous film into a conduit in a closed system, and even a mesh can be used in the closed system.

In the culture method of pluripotent stem cells described in patent document 1, a mesh made by knitting nylon wire or metal wire is used as a specific method for dividing large cell aggregates, and large cell aggregates are passed through the mesh to be divided into small cell aggregates corresponding to the mesh-holes (square pass holes) of the mesh.

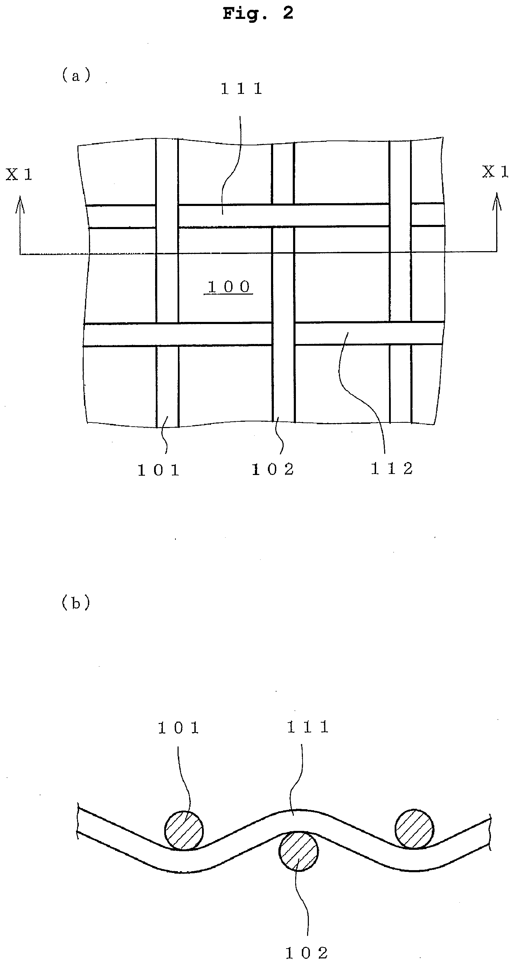

However, when the present inventors have examined in detail the division of the cell aggregates using the mesh as described above, it was found that the cell aggregates passing through the mesh may not be preferably divided due to the structure peculiar to the mesh. The mesh is a kind of sheet-like material, and when the sheet surface is seen macroscopically in a straight view, the warp wire and the weft wire appear to intersect linearly as shown in Fig. 2(a), and the mesh-hole also looks like a flat plane square. However, when each mesh-hole is microscopically observed, since the warp wire and the weft wire are knitted three-dimensionally so as to avoid each other, four wires (two warp wires (101, 102) and two weft wires (111, 112)) constituting four sides surrounding one square mesh-hole 100 wave broadly in the thickness direction of the mesh as shown in Fig. 2(b).

When cell aggregates pass through such mesh-hole surrounded by four wavy wires, since the cross-sectional shape of each wire is a circular shape, and the surface of the wire body is a curved surface, the cell aggregates may not be divided appropriately or sharply in some cases. When a thinner wire is used to improve such defect of the mesh, the strength of the mesh is reduced. When this is improved by increasing the wire strength, the mesh becomes more expensive. When cell aggregates are divided by a mesh formed using a wire and the flow velocity of a liquid medium is low, the cell aggregates cannot be cut but are only trapped in the mesh of the net. As a result, dividing is not appropriately performed and the collection rate of the cell aggregates becomes low. Therefore, a certain level of high flow velocity is necessary. On the other hand, when the flow velocity of a liquid medium is high, the divided cell aggregates receive a shear due to the high-speed flow and become smaller. It is not preferable to divide cell aggregates of pluripotent stem cells to have an outer diameter of 40 µm or less, since the cells are significantly damaged as evidenced by apoptosis of the cell and the like.

As described above, when cell aggregates are divided using a conventional mesh, a low flow velocity of a liquid medium leads to a low cell recovery rate and a high flow velocity of a liquid medium leads to large damage on the cell aggregates and low expansion culture efficiency.

Thus, the present invention proposes use of a porous film in which a large number of through-holes are arranged in a predetermined region on the main surface of the film as a mesh structure. Preferred embodiments of the porous film are described in detail in the following with reference to Examples.

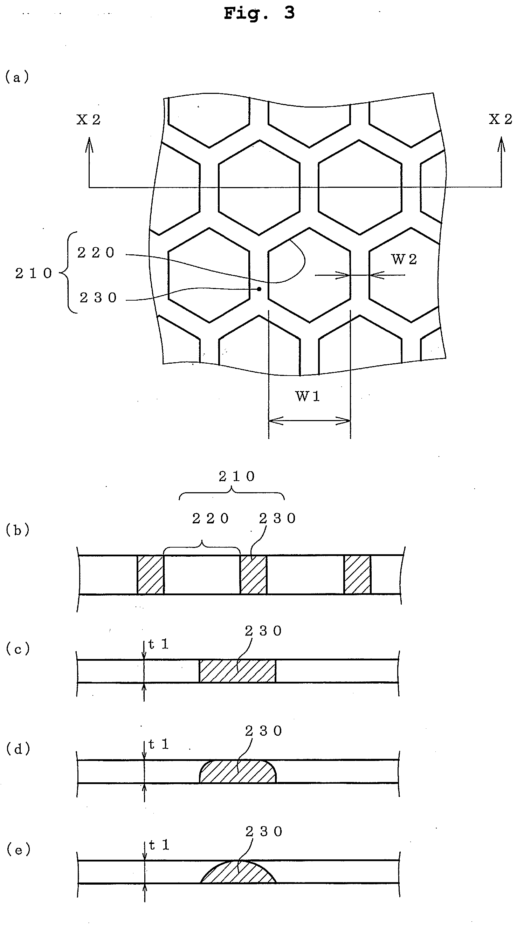

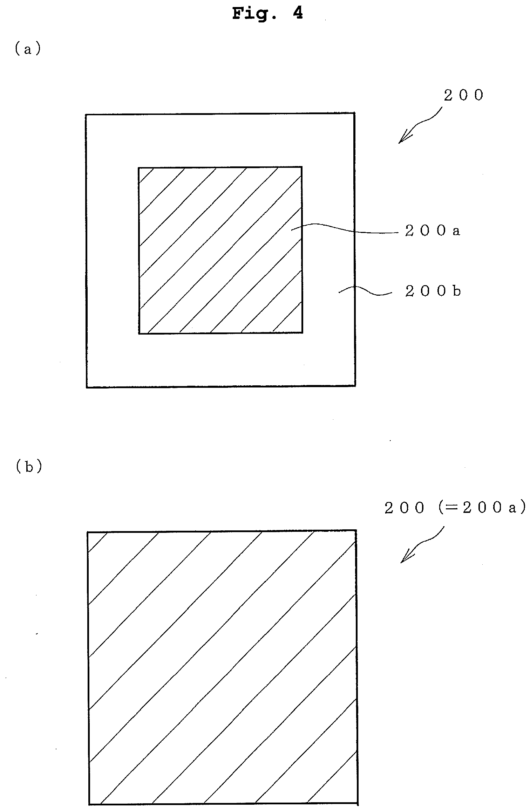

The porous film has a film-like main body part. A highly rigid frame, tab, or the like may be further provided on the outer peripheral edge portion or the like of the main body part to improve handleability. In the embodiments described below, the entire porous film is a film-like main body part, and thus the entire device is a single film. As shown in Fig. 3(a), a predetermined region on the film surface of the main body part of the porous film has a mesh structure 210 in which a large number of through-holes 220 are arranged on the film surface. The predetermined region on the film surface may be a part or all of the film surface. For example, in the case of Fig. 4(a), an outer circumference region 200b of a film-like main body part 200 is a flat film free of a through-hole, a through-hole is provided in the center region 200a. When the visible outline defining the outer circumference of the mesh structure (e.g., the boundary line of the outer circumference of the central region 200a) crosses the opening of the through-hole, the opening of the through-hole is a small opening only inside the visible outline. In this case, the through-hole of the small opening may be provided as it is, or the through-hole of such a small opening may not be provided, or a through-hole may be provided so that the opening with the original shape exceeds the visible outline.

### (Mesh structure)

As shown in Fig. 3(a), a mesh structure 210 of the porous film is composed of a large number of through-holes 20 penetrating the aforementioned predetermined region in the direction of the film thickness, and a beam part 230 which is a partition between the through-holes. The through-hole 220 has an opening shape large enough to allow the cell aggregate after division to pass through. On the other hand, the beam part 230 is a remainder obtained by subtracting the aforementioned through-hole from the main body part in the aforementioned predetermined region. The beam part functions as a part for cutting the cell aggregate to be divided, and are integrally connected so as to form a network. With this constitution, the problem of the aforementioned mesh is suppressed, and a large-grown cell aggregate can be preferably divided.

In a porous film usable in the present invention, as mentioned above, a large number of through-holes are arranged on the film surface, and a predetermined region (a part or all of the region) of the film surface has a mesh structure. This mesh structure is a kind of porous film composed of through-holes that function as mesh-holes and beam parts that function as partition parts between adjacent through-holes. In the region of this mesh structure, as shown in Fig. 3(a), the beam part 230 is two-dimensionally connected as a net as the remainder of the film excluding the through-holes, and there is no waviness of mesh wires. Therefore, the cell aggregate that collides with such a beam part free of waving can be more preferably divided than the wavy mesh wires described above. Furthermore, as shown in Fig. 3(b) or Fig. 3(c), the cross-sectional shape of the beam part 230 is not a circle but close to a quadrangle or a rectangle (rectangle with long side in thickness direction, rectangle with short side in thickness direction, or square). Therefore, the edge of the beam part 230 at the opening of each through-hole can divide the cell aggregate sharply without resistance, and thus the damage to the cell aggregate at the time of cutting may be smaller in some cases.

When dividing cell aggregates by using the porous film, therefore, even when the flow velocity of the liquid passing through the net-like region (such as a liquid medium in which the cell aggregates to be divided are dispersed) is set higher than in the case of division using a conventional mesh, crushing of the cell aggregate into excessively fine cell aggregates can also be suppressed.

The cross-sectional shape of the beam part (the shape of the cross section perpendicular to the longitudinal direction of the beam part) can be rectangular (right-angled quadrilateral) depending on the relationship between the width W2 of the beam part and the thickness t1 of the porous film, as shown in Fig. 3(b) or Fig. 3(c).

The rounded shape of the two corners on the inlet side of the rectangle, as shown in Fig. 3(d) or Fig. 3(e), has a higher survival rate of the divided cell aggregate and may be preferable in some cases. It is considered that this is because the damage to the cells is reduced, the individual cells themselves are less likely to be cut, and the cells are more likely to be separated at the interface where the cells adhere to each other, compared to the case where the two corners on the inlet side are sharp right-angled edges.

In the embodiment of Fig. 3(d), only the two corners on the rectangular inlet side have a locally rounded shape, and therefore, a flat plane remains on the inlet side surface of the beam part. On the other hand, in the embodiment of Fig. 3(e), the two corners on the rectangular inlet side is more rounded than the embodiment of Fig. 3(d), and the entire cross-sectional shape is semicircular (more precisely, a shape with circular arc and chord). Therefore, the surface on the inlet side of the beam part is entirely curved as if it were the surface of a cylindrical body. As mentioned above, to reduce the damage to the cell, a larger radius of the roundness of the corner is preferable, and the embodiment of Fig. 3(e) is more preferable than the embodiment of Fig. 3(d). The radius of the roundness also varies depending on the width W2 of the beam part and the thickness t1 of the porous film, and is, for example, about 1 µm - 100 µm. The radius of the roundness may be uniform like a circular arc, or may vary from place to place.

In a preferred embodiment of the porous film usable in the present invention, the opening shape of the through-hole is a shape closer to a circle (e.g., square or regular hexagon), through-holes with the same opening shape are arranged in a matrix or close-packing shape, and the width of the beam part is uniform. As a result, the damage to the cell aggregate at the time of cutting can be made smaller, and a sphere-shaped preferable cell aggregate having a preferable size can be obtained.

In the porous film usable in the present invention, it is relatively easy in processing to narrow the width of the beam part. When the opening shape is a regular hexagon, the strength of the entire mesh structure is high, and the width of the beam part can be further narrowed. Since the cell aggregate can be further divided without resistance and the opening ratio (the ratio of the opening area to the unit area of the region of mesh structure) can be increased, the above-mentioned problem of mesh can be solved.

### (Size limitation on opening shape of through-hole of porous film)

The equivalent-circle-diameter of the opening shape of the through-hole varies depending on the type of cells constituting the cell aggregate to be divided. For example, when the cell is a pluripotent stem cell, an embryonic stem cell, or the like, it is about 40 - 90 µm, more preferably 50 - 80 µm, further preferably 60 - 70 µm. In the following, preferable size and the like of each part are illustrated for cases where the cell is a pluripotent stem cell, an embryonic stem cell or the like, but other cells may also be changed to have appropriate sizes.

With only the above-mentioned definition of the aforementioned equivalent-circle-diameter, an elongated opening shape such as a slit and an intricate opening shape such as a maze are also included. Therefore, in the present invention, in addition to the aforementioned limitation on the equivalent-circle-diameter, the opening shape being a shape capable of accommodating a circle having a diameter of 35 µm to 85 µm (hereinafter referred to as contained circle) is added to the limiting condition. Here, the opening shape being able to accommodate the contained circle also includes the case where the contained circle is inscribed in the opening shape and the case where the contained circle matches the opening shape.

When shapes having the same equivalent-circle-diameter are compared, regular hexagon has a larger diameter of the contained circle than a square. In the case of a square corresponding to the preferred lower limit of the equivalent-circle-diameter of 40 µm, the length of one side of such square is about 35.449 µm, in which case the diameter of the contained circle is about 35.449 µm or less. Therefore, in the present invention, 35 µm is set as a preferable lower limit of the diameter of the contained circle. In the case of a regular hexagon corresponding to 90 µm, which is a preferable upper limit of the equivalent-circle-diameter, the distance between two opposing sides in such regular hexagon is about 85.708 µm, and the diameter of the contained circle in this case is about 85.708 µm or less. Therefore, in the present invention, 85 µm is set as a preferable upper limit of the diameter of the contained circle.

The diameter of the aforementioned contained circle is more preferably 44 µm - 76 µm, further preferably 53 µm - 67 µm. When the opening shape is circular, the diameter of the contained circle = equivalent-circle-diameter, otherwise, the diameter of the contained circle <equivalent-circle-diameter.

### (Opening shape of through-hole of porous film)

The opening shape of a large number of through-holes provided in the mesh structure is not particularly limited, and may be circular, elliptical, triangular, quadrangular, hexagonal, or other polygonal or irregular shape. When it is a shape with an acute-angled internal angle such as triangle and the like, the opening ratio cannot be increased from the viewpoint of film strength, and problems of decrease in the collection rate and the like may occur. In the case of a circular shape, since the width of the beam part is not constant and the area where the beam parts are connected to each other is large, the cutting property of the cell aggregate by the beam part is not preferable. In contrast, in the case of a regular hexagon, since the internal angle is an obtuse angle and the area where the beam parts are connected to each other is small, the aforementioned problems are preferably suppressed.

To obtain a uniform cell aggregate after cutting, it is preferable that all opening shapes are congruent with each other.

The width of the beam part (distance between the through-holes adjacent to each other) is preferably uniform because the cuttability along the length of the beam part becomes uniform.

From these aspects, the opening shape of the through-hole is preferably quadrangle or hexagon, and square and regular hexagon with sides (beam part) around the opening that are equal to each other are more preferable. A regular hexagon is a preferable shape since it is closer to a circle.

### (Configuration pattern of opening of porous film)

When all the opening shapes are congruent regular hexagons, the arrangement pattern of the openings on the film surface is preferably a close-packing shape, in which case the beam parts are in a honeycomb shape connected to each other to form a net as shown in Fig. 3(a). This embodiment is preferable because width W2 of all the beam parts is uniform except for the portion where the terminal portions of the three beam parts are connected to each other.

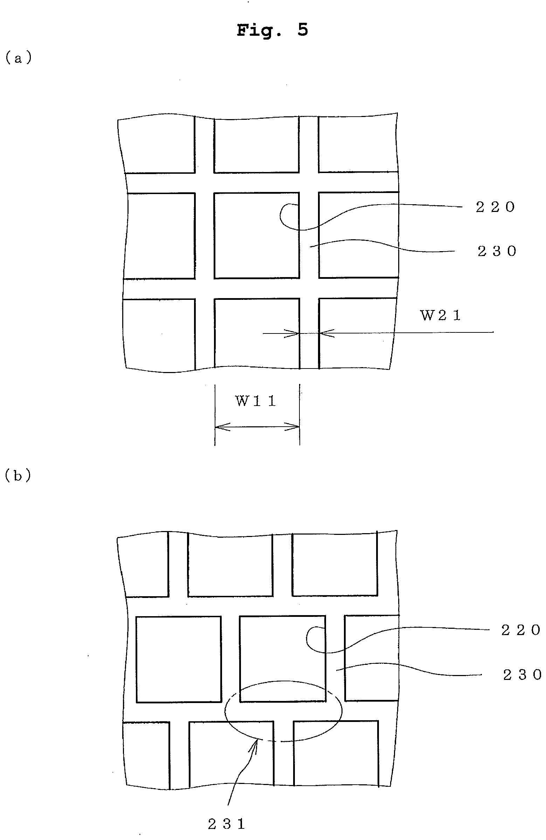

When all the opening shapes are congruent squares, the arrangement pattern of the openings on the film surface is preferably a square matrix, in which case the beam parts are arranged in an orthogonal lattice mesh connected to each other as shown in Fig. 5(a). This embodiment is also preferable because width W21 of all the beam parts is uniform except for the portion where the terminal portions of the four beam parts are connected to each other. As shown in Fig. 5(b), it may be an arrangement pattern in which the square openings are laterally offset every other row. Since the beam part in the lateral direction is divided into two like part 231 surrounded by the dashed line, the cuttability is different from that of the orthogonal grid as shown in Fig. 5(a).

As. shown in Fig. 3(a), when the openings of the regular hexagon are arranged in a close-packing shape, of the six sides of the regular hexagon, the distance W1 between two parallel sides facing each other may be the diameter of the aforementioned contained circle. It is preferably 38 µm - 85 µm, more preferably 48 µm - 76 µm, further preferably 57 µm - 67 µm. In this case, the width W2 of the beam part is preferably 10 µm - 60 µm, more preferably 20 µm - 40 µm.

As shown in Fig. 5(a), when the openings of the square are arranged in a square matrix shape, of the four sides of the square, the distance W11 between two parallel sides facing each other may be the diameter of the aforementioned contained circle. It is preferably 35 µm - 80 µm, more preferably 44 µm - 71 µm, further preferably 53 µm - 62 µm. In this case, the width W21 of the beam part is preferably 10 µm - 60 µm, more preferably 20 µm - 40 µm.

### (Thickness of main body part)

While the thickness of the film-like main body part is not particularly limited, to make the beam part a thin line, it is preferably 10 µm - 60 µm, more preferably 20 µm - 40 µm.

### (Material of main body part)

The material of the film-like main body part is not particularly limited, and metal materials such as gold, silver, copper, iron, zinc, platinum, nickel, chrome, palladium and the like, and alloys consisting of any combination of these materials can be mentioned. Preferred alloy is, for example, stainless steel, brass or the like.

### (Production method of porous film)

The production method of the porous film is not particularly limited and a suitable method according to the material such as resin form, punching out, LIGA (Lithographie Galvanoformung Abformung) and the like can be selected. Since the opening shape and the width of the beam part are minute, a production method using LIGA is exemplified. In the production method using LIGA, for example, a metal mold for electrocasting is created by lithography, and an electrochemical reaction is used in the electrocasting tank to form a metal plating layer to be the porous film on the surface of the metal mold, and the metal plating layer is peeled off from the mold and used as the porous film.

A shape in which the two corners on the inlet side of the cross-sectional shape of the beam part are rounded as shown in Figs. 3(d), (e) can also be obtained by, for example, changing the shape of the concave portion of the aforementioned metal mold to a shape with rounded inside-corners.

Where necessary, two or more mesh structures arranged in series may be used. For example, two or more mesh structures may be arranged in a stack in the holder to be explained below, or two or more holders containing one mesh structure and connected in series may be used. The specifications of the mesh structure when two or more are used may be different from each other or may be the same.

### (Holder for preferably using the mesh structure)

In the present invention, a holder for preferably using the mesh structure (mesh or porous film) is proposed. By setting the mesh structure in the holder, a preferable divider is configured. The holder not only allows the cell aggregate to be divided to preferably pass through the mesh structure together with the liquid, but also makes it possible to continuously perform cell culture, cell aggregate division, and subculture of the cell aggregate after division in a closed system.

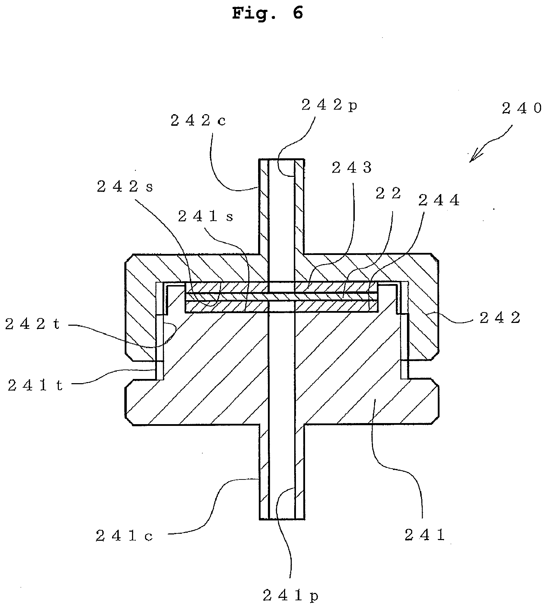

Fig. 6 is a cross-sectional view showing one embodiment of the structure of the aforementioned holder. As shown in the figure, a holder 240 is composed of a holder body 241 having a configuration surface 241s for arranging the mesh structure 22, and a cap part 242 that covers the mesh structure 22 arranged on the configuration surface 241s and detachably fixed to the holder main body. In the embodiment of Fig. 6, the mesh structure (porous film) 22 is sandwiched between two pieces of gaskets (sheet-shaped sealing members) 243 and 244, whereby the liquid medium is sealed so as not to leak out of the holder. The inner surface of the cap part 242 is a pressing surface 242s for pressing the mesh structure 22 against the holder main body 241.

The holder main body 241 has a first through-hole 241p that opens in the configuration surface 241s, and the cap part 242 has a second through-hole 242p that opens in a pressing surface 242s. These first through-hole and second through-hole function as dividing conduits. As shown in Fig. 6, when the cap part 242 is fixed to the holder main body 241, the center of the opening of the first through-hole 41p and the center of the opening of the second through-hole 42p coincide with each other. Unlike the conventional mesh, since the front and back surfaces of the porous film 22 is flat, the liquid medium can be easily and preferably air-tightly sandwiched so that the liquid medium may not leak in the transversely direction.

The outer shape of the gaskets 243, 244 is preferably equal to or larger than the outer shape of the mesh structure. The materials of the gaskets 243 and 244 may be, for example, silicon or the like, which shows preferable sealability without affecting the living body.

In the embodiment of Fig. 6, at the center of each of the two pieces of gasket, a circular through-hole having the same cross-sectional shape as the first through-hole 241p and the second through-hole 242p is provided. These through-holes are contained in the dividing conduit. In the embodiment of the figure, the inner diameter of these through-holes is about 1.6 mm and the through-holes are aligned on a line. As a result, the flow of the liquid medium passes through the mesh structure in the cross-sectional shape of these through-holes. If the flow changes as it passes through the mesh structure, the divided cell aggregate may also be subjected to the shearing force of the flow, and may become smaller. Therefore, the inner diameter of the first through-hole 241p, the second through-hole 242p, and the through-hole of each of the two pieces of gasket is preferably the same. However, the inner diameters of these through-holes may be different from each other as long as they do not cause a turbulent flow that gives an adverse influence.

In the embodiment of Fig. 6, the inner diameter of the conduit of the first through-hole 241p, the second through-hole 242p is constant, and the ratio of the inner diameter (D1) of the conduit to the inner diameter (d1) of the through-hole of the gasket that determines the effective diameter of the flow passing through the mesh structure, (D1/d1), is preferably about 0.33 - 3, more preferably 0.5 - 2, further preferably about 1. In a preferred embodiment, D1/d1=1. In consideration of matching the central axis of the flow path, appropriate allowable sizes may be given to the inner diameter of each through-hole, the outer diameter of the gasket, the diameter of the configuration surface 241s, and the like.

In the embodiment of Fig. 6, a male screw 241t is provided on the outer circumference of the body of the holder main body 241, and a female screw 242t is provided on the inner surface of the body of the cap part 242. These screws screw and fix the holder main body 241 into the cap part 242 and the mesh structure 22 is sandwiched and pressed to prevent leaking. A knurl, a head portion of a hexagon head bolt, or the like for exerting a force for rotating (or holding) these may be provided on the outer circumference of each body of the holder main body 41 and the cap part 242.

The attachment/detachment structure between the holder main body and the cap part is not limited to the aforementioned screw structure described above, and may be a one-touch coupling structure or a structure in which the cap part is tightened to the holder main body by using bolts and female screws, or the like.

The material of the holder is not particularly limited, and examples thereof include organic polymer materials such as polystyrene, polypropylene, poly(ethylene terephthalate), polycarbonate, acrylic, silicon, polyvinylidene fluoride and the like, and metal materials such as stainless steel and the like. From the viewpoint of molding at a low cost and resistance to autoclaves and gamma rays, polypropylene, polycarbonate, and acrylic are exemplified as preferable materials.

The inner diameters of the conduits 241p and 242p (the cross-sectional shape is circular) of the holder main body 241 and the cap part 242, respectively, are not particularly limited and may be appropriately determined according to the scale and flow rate of the cell culture system. About 0.5 mm - 15 mm is versatile and useful. In the embodiment of Fig. 6, the inner diameter of the conduit is 1.6 mm.

To connect these pipe lines to external pipes, connecting pipes 241c and 242c protrude respectively from the holder body main body and cap part. The outer surface of the body of these pipes may be, for example, in the shape of a hose nipple (also called "bamb fitting joint"), or may be press-fitted into a soft tube or the like (or a soft tube or the like may be press-fitted) to form connection. It may be a structure having connectivity with a known connector such as a female side or a male side of a known one-touch joint (quick coupling), a push-in joint for a resin tube, a tightening joint, or the like.

The outer shape of the holder is not particularly limited. In the embodiment of Fig. 6, the overall outer shape (excluding the pipe portion) of the holder is cylindrical and has a diameter of 20 mm. The total length is about 20 - 25 mm. These numerical values are examples, and may be appropriately determined according to the inner diameter of the conduit, the structure of the connector, and the like.

By using the holder as described above, the loss of cells remaining (trapped) in the mesh structure does not occur, turbulent flow is less likely developed as the cell aggregate passes through the mesh structure with the liquid, movement along the layer flow allows the cell aggregate to be cut without resistance, damage to cells is reduced, and the survival rate of the cell aggregate after division can be improved.

Where necessary, two or more of the mesh structures arranged in series may be used. For example, two or more of the mesh structures may be arranged in a stack in one holder, or two or more holders connected in series may be used. The specifications of the mesh structures when two or more are used may be different from each other or may be the same.

### (Cell aggregate)

The type of cells constituting the cell aggregate to be cultured by the cell culture system (i.e., cell aggregate produced by the production method of the present invention) is not particularly limited as long as it is a cell that forms a cell aggregate (also referred to as "spheroid") by suspension culture, and any cell can be used. The cells constituting such cell aggregate can be animal or plant-derived cells, and are particularly preferably derived from animal cells. As an animal species from which such cells are derived, mammals such as rat, mouse, rabbit, guinea pig, squirrel, hamster, vole, platypus, dolphin, whale, dog, cat, goat, bovine, horse, sheep, swine, elephant, common marmoset, squirrel monkey, Macaca mulatta, chimpanzee and human and the like are more preferable. The cells constituting the cell aggregate may be those established as cultured cells or primary cells obtained from biological tissues. Further, the cells constituting the cell aggregate may be pluripotent stem cells, which include embryonic stem cells (ES cells), induced pluripotent stem cells (iPS cells), mesenchymal stem cells, neural stem cells and the like. The cells constituting the cell aggregate may be differentiated cells such as hepatocytes, pancreatic islet cells, kidney cells, nerve cells, corneal endothelial cells, chondrocytes, myocardial cells and the like. Furthermore, the cells constituting the cell aggregate may be cells induced to differentiate from umbilical cord blood, bone marrow, fat, and blood-derived tissue stem cells, or tumorigenic cells, cells transformed by genetic engineering techniques, or cells infected with viral vectors. In one embodiment of the present invention, the cell constituting the cell aggregate is preferably a human-derived pluripotent stem cell, especially human iPS cell. In the present specification, the "suspension culture" means culturing cells or cell aggregates (that is, cell clumps having a three-dimensional structure (spherical or cluster of grapes) formed by a large number of cells) under conditions free of adhesion to an incubator.

In one embodiment, the cell seeding density at the time of suspension culture of cells or cell aggregates in the first container in the cell culture system of the present invention is not particularly limited as long as a desired effect is obtained. It may be generally 1×10³ - 1×10⁷ cells/mL, preferably 1×10⁴ - 1×10⁶ cells/mL, more preferably 5×10⁴ - 1×10⁶ cells/mL, further preferably 1×10⁵ - 1×10⁶ cells/mL, further more preferably 1×10⁵ - 8×10⁵ cells/mL, particularly preferably 1.0×10⁵ - 3.0×10⁵ cells/mL.

### (Size of cell aggregate to be divided)

The outer diameter of the cell aggregate to be divided is not particularly limited. When the cell is a pluripotent stem cell, an embryonic stem cell, or the like, 50 µm - 300 µm is preferable, 100 µm - 200 µm is more preferable, and 120 µm - 180 µm is further preferable.

For the outer diameter of the cell aggregate, the area of the cell aggregate image obtained by a microscope (including an electron microscope and an optical microscope) is measured, and the diameter of a circle having an area the same as the area (equivalent-circle-diameter) can be adopted.

The cell aggregate having the aforementioned outer diameter is divided by a divider to have an outer diameter of about 40 µm - 120 µm, more preferably about 50 µm - 90 µm.

The outer diameter (equivalent-circle-diameter) of the divided cell aggregate does not always match the equivalent-circle-diameter of the opening shape of the through-hole in the mesh structure of the divider, and may be larger or smaller than the equivalent-circle-diameter of the opening shape. For example, a cell aggregate formed into a long columnar shape by passing through a through-hole may have an equivalent-circle-diameter larger than the equivalent-circle-diameter of the opening shape depending on the observation angle. A small cell aggregate that did not completely fill the through-hole and passed through same while creating a gap with the inner wall of the through-hole (wall surface of the beam part) may have an equivalent-circle-diameter smaller than the equivalent-circle-diameter of the opening shape.

### (Liquid medium)

The liquid medium that can be used for the aforementioned cell culture is not particularly limited, and includes a medium suitable for the cells to be cultured and that can form a cell aggregate as a result of culturing the cells in a floating state. As such medium, a liquid medium containing fine structures for suspending cells or cell aggregates therein in which the fine structures are dispersed and floating therein is preferable and, for example, a medium capable of sphere culture and a medium containing a specific polysaccharide, and a medium containing a specific polysaccharide is more preferable from the viewpoint of cell culture efficiency and the like (see WO2014/017513 for the detail) can be mentioned. Examples of the polysaccharide contained in such a medium include deacylated gellan gum, daiyutan gum, carrageenan and xanthan gum, and salts thereof, and deacylated gellan gum is preferable. By adding such a polysaccharide to a known medium, a liquid medium that can be used for the aforementioned cell culture can be easily prepared. Examples of the known medium that can be used when the cell is derived from an animal include Dulbecco's Modified Eagle's Medium (DMEM), hamF12 medium (Ham's Nutrient Mixture F12), DMEM/F12 medium, McCoy's 5A medium, Eagle MEM medium (Eagle's Minimum Essential Medium; EMEM), αMEM medium (alpha Modified Eagle's Minimum Essential Medium; αMEM), MEM medium (Minimum Essential Medium), RPMI1640 medium, Iscove's Modified Dulbecco's Medium (IMDM), MCDB131 medium, William medium E, IPL41 medium, Fischer's medium, StemPro34 (manufactured by Invitrogen), X-VIVO 10 (manufactured by Cambrex Corporation), X-VIVO 15 (manufactured by Cambrex Corporation), HPGM (manufactured by Cambrex Corporation), StemSpan H3000 (manufactured by STEMCELL Technologies), StemSpanSFEM (manufactured by STEMCELL Technologies), StemlineII (manufactured by Sigma Aldrich), QBSF-60 (manufactured by Qualitybiological), StemProhESCSFM (manufactured by Invitrogen), Essential8 (registered trade mark) medium (manufactured by Gibco), Essential8 (registered trade mark) Flex medium (manufactured by Thermo Fisher), StemFlex medium (manufactured by Thermo Fisher), mTeSR (registered trade mark) 1 or 2 or Plus medium (manufactured by STEMCELL Technologies), REPRO FF or REPRO FF2 (manufactured by REPROCELL), PSGro hESC/iPSC medium (manufactured by System Bioscience), NutriStem (registered trade mark) medium (manufactured by Biological Industries), CSTI-7 medium (manufactured by Cell Science & Technology Institute), MesenPRO RS medium (manufactured by Gibco), MF-Medium (registered trade mark) mesenchymal stem cellular proliferation medium (manufactured by TOYOBO), Sf-900II (manufactured by in Invitrogen), Opti-Pro (manufactured by Invitrogen), StemFit (registered trade mark) AK02N or Basic02 or AK03N or Basic03 or Basic04 medium (manufactured by AJINOMOTO HEALTHY SUPPLY), STEMUP medium (manufactured by Nissan Chemical Corporation.) and the like. FCeM (registered trade mark) medium (manufactured by Nissan Chemical Corporation) can be used preferably since it contains polysaccharides that can uniformly disperse cell aggregates.

When a structure for suspending a cell aggregate (the above-mentioned deacylated gellan gum and the like) is dispersed and suspended in a liquid medium and when such structure passes through a divider together with the cell aggregate, the structure is partly disintegrated, and the property of maintaining the cell aggregate in a suspended state decreases.

### (Liquid feeding device and piping)

The cell culture system is used together with a liquid feeding device F1 for sending a liquid medium in which cell aggregates are dispersed. The liquid feeding device F1 may be an element constituting the cell culture system, or may be an external apparatus that does not belong to the cell culture system but is used when the cell culture system is activated. The position of the liquid feeding device F1 is not particularly limited and may be on the conduit P1 or conduit P2 in the configuration of, for example, Fig. 1.

A liquid feeding device usable for the cell culture system is not particularly limited, and peristaltic pumps such as a tube pump (also called roller pump) and syringe pumps are preferable. In particular, a peristaltic pump is preferable since closed piping can be easily constructed. In addition, each container itself may have a structure capable of delivering the liquid in the inside like a syringe.

The peristaltic pump is a pump that moves the liquid in the set pumping tube by moving the position at which the elastic and flexible pumping tube is crushed in the feed direction. Even with a peristaltic pump, a large number of cell aggregates are preferably delivered with the liquid medium without being crushed. For more information on the feed structure of the peristaltic pump, prior art can be referred to. When a peristaltic pump is used, the tube for piping used for a conduit preferably has a part that can be attached to the peristaltic pump as a pumping tube and has a shape, flexibility and elasticity permitting function and operation as a pumping tube.

The connector and coupling for piping are not particularly limited, and it is preferable to use a connector that can be connected aseptically, such as a sterile connector and the like.

### (Constitution for washing divider)

The present inventors have found that when the divider is continuously used for the division of cell aggregate, solid components such as debris of cell aggregates and fine structures contained in the medium are deposited on the beam part (or wire) of the mesh structure of the divider and, along with the deposition, the effective area of the mesh structure of the divider (the total area of openings through which a liquid can pass) gradually decreases, as a result of which the cell aggregate is subject to shear by the high-speed flow and damaged, and divided into small cell aggregates, thereby possibly reducing the survival rate.

Therefore, it is preferable to periodically replace the divider or mesh structure in the cell culture system with one in which debris of cell aggregates or the like is not deposited.

On the contrary, the present inventors have found that debris of cell aggregates clinging to the beam part of the mesh structure is removed and a decrease in the effective area of the mesh structure is suppressed by flowing a predetermined liquid (such as a liquid medium containing a cell aggregate and a liquid exclusive for washing, which are described later) backward every time a predetermined amount of a liquid containing a cell aggregate passes through the mesh structure. That is, it was found that a decrease in the effective area of the mesh structure can be suppressed by passing a predetermined liquid through the mesh structure in the direction opposite to that at the time of division, as a result of which a decrease in the survival rate of cells contained in the divided cell aggregate can be suppressed. In the following, a predetermined liquid backward flowing process performed to suppress a decrease in the effective area of the net structure is called "backflow washing of the net structure".

Periodic backflow washing of the mesh structure reduces the number of exchange of the divider and the mesh structure and thus suppresses the decline in the cutting performance of the mesh structure while maintaining a closed system.

In a preferred embodiment of the cell culture system, to enable backflow washing of the mesh structure (i.e. such that the liquid for suspending or washing passes through the mesh structure in a direction opposite from the direction of the suspension containing the cell aggregate to be divided that passes through the aforementioned mesh structure), a backward direction feed function or backward direction feed apparatus for moving a liquid for the suspension or washing may be further provided.

The aforementioned backward direction feed function may utilize the backflow function of the above-mentioned liquid feed apparatus provided in the cell culture system, or the backflow function may be further added to the liquid feed apparatus. The backflow function of the liquid feed apparatus may be, for example, reverse rotation of peristaltic pump, reverse operation of syringe pump (suction against extrusion), pressing of a flexible container, and the like.

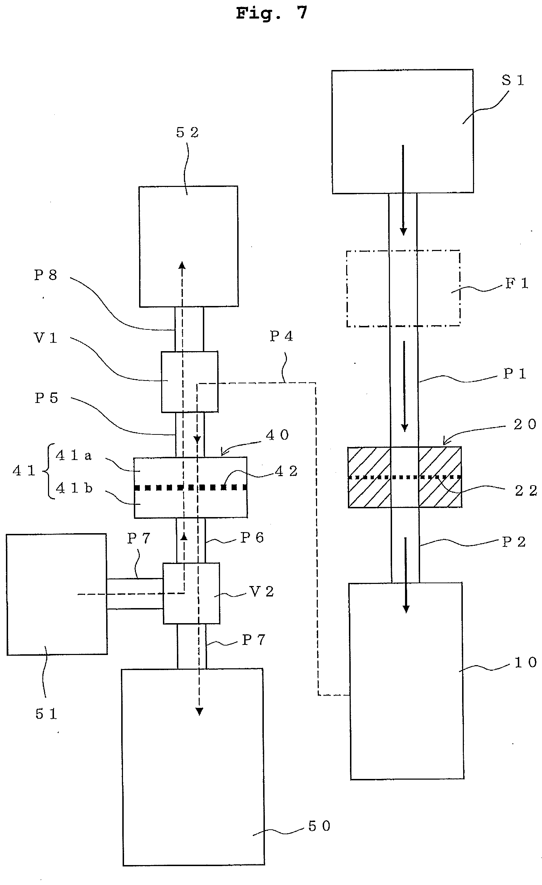

In addition, the backward direction feed apparatus to perform backflow washing of the mesh structure and the piping configuration thereof are not particularly limited. For example, in the constitution relating to the collector in Fig. 7, switching valves V1 and V2 are operated such that a new liquid medium flows from the liquid supply container 51 through conduit P7, conduit P6, collector 40, conduit P5, and conduit P8 in this order, and enters collection container 52. By this operation, the new liquid medium passes through the collector 40 in the backward direction. The constitution for such backflow is also applied to the backflow washing of the mesh structure, and a switching valve, a fluid supply container, and the like are added as appropriate, and a constitution for flowing a liquid backward to perform backflow washing of the mesh structure can be appropriately constructed.

By the aforementioned constitution and operation thereof, a suspension containing a cell aggregate or a new liquid medium passes through the mesh structure in the opposite direction, and the cell aggregate and the like entangled with the beam part of the mesh structure are removed from the beam part.

### (Predetermined liquid to be flown backward in backflow washing of mesh structure)

The "predetermined liquid" to be flown backward in backflow washing of the mesh structure is not particularly limited, and a liquid that enables the mesh structure to be used continuously can be mentioned. For example, a liquid immediately after passing through the mesh structure (i.e., liquid medium (suspension) containing divided cell aggregates), a liquid medium (not including cell aggregate) similar to the liquid medium used when dividing the cell aggregate, a liquid from which the cell aggregate and fine structures for suspending cells have been removed from the liquid medium used when dividing the cell aggregate, and the like can be mentioned.

### (Embodiment example of backflow washing of mesh structure)

The embodiment of backflow washing of the mesh structure is not particularly limited and includes the following.
(i) An embodiment in which a liquid medium containing the cell aggregate to be divided passes through the mesh structure and then the flow is reversed, whereby the liquid medium containing the divided cell aggregate passes through the mesh structure in the backward direction.
(ii) An embodiment in which a liquid medium containing the cell aggregate to be divided passes through the mesh structure, the flow path is switched, a liquid (liquid medium, etc.) not containing a cell aggregate or the like is supplied to the downstream side (outlet side) of the mesh structure, and the flow is reversed, whereby the liquid medium not containing a cell aggregate or the like passes through the mesh structure in the backward direction.
(iii) An embodiment in which a liquid medium containing the cell aggregate to be divided passes through the mesh structure, immediately thereafter a liquid medium not containing a cell aggregate or the like is flown in the same direction, and the flow is reversed after the liquid medium has passed through the mesh structure, whereby the liquid medium not containing a cell aggregate or the like passes through the mesh structure in the backward direction.

The frequency of backflow washing of the mesh structure is not particularly limited, and may be every one division, or every two or more divisions, and can be appropriately determined according to the total number of cell aggregates that have passed through a unit area of the mesh structure, or by comprehensively considering the benefit of suppressing a decrease in the survival rate of cells contained in the divided cell aggregate and the disadvantage of labor of backflow washing of the mesh structure and the expansion of the system. Preliminary experiments can determine how many cell aggregates that pass through a unit area of the mesh structure reduce how much the cutting performance of the mesh structure.

The flow velocity and cleaning time of the aforementioned predetermined liquid when performing backflow washing of the mesh structure can be appropriately determined according to the kind of the liquid and the effect of the backflow washing. The flow velocity is not particularly limited and is, for example, about 10 mm/sec - 500 mm/sec, particularly preferably about 50 mm/sec - 300 mm/sec. As the flow velocity, the flow velocity at which the liquid (suspension) enters the divider (or exits the divider) can be adopted. The flow velocity can be obtained based on the operation of extruding or sucking a predetermined amount of solution at a constant speed in a predetermined time using a liquid feed pump such as a syringe and the like. In addition, the flow velocity when the liquid passes through the mesh of the mesh structure can be calculated by dividing the flow rate by the aforementioned liquid feed pump by the total opening area of the mesh. The washing time (backflow time) is also not particularly limited, and when the flow velocity of the liquid is within the aforementioned range, it is about 0.1 sec - 5 sec, particularly preferably, about 0.3 sec - 2 sec.

### (Collector)

In a preferred embodiment of the cell culture system, a collector is further provided for collecting a cell aggregate grown to a predetermined size by division and culture. Fig. 7 shows one embodiment in which a collector is added to the constitution shown in Fig. 1. In the example of Fig. 7, a collector 40 is connected to a first container 10 via a conduit P4 so as to receive the cell aggregate and the liquid medium suspension cultured in the first container 10 without contact with the outside air. It is preferable to provide an open-close valve at the connecting part between the first container 10 and the conduit P4.

As shown in Fig. 7, the collector 40 has a separation chamber 41, the separation chamber is divided into a first chamber 41a and a second chamber 41b by a mesh structure for separation 42 for separating the liquid medium and the cell aggregate, and an internal flow path is configured such that the liquid medium that entered the collector 40 moves from the first chamber 41a through the mesh structure for separation 42 to the second chamber 41b and goes out of the collector 40. To maintain the closed system, a waste liquid container 50 for receiving the liquid medium is preferably connected to the subsequent stage of the collector 40. The collected cell aggregate can be washed with phosphate buffer (PBS) or a desired medium.

### (Mesh structure for separation)

The mesh structure for separation 42 has a mesh-hole with a size predetermined to prevent a cell aggregate of a size intended to be collected from passing through (that is, being trapped). As a result, among the cell aggregates that have entered the collector together with the liquid medium, the cell aggregate of a size intended for collection cannot pass through the mesh structure for separation 42 and stays in the first chamber 41a. Thus, the cell aggregate remaining in the first chamber 41a can be collected.

The mesh structure for separation may be a mesh or a porous film, similar to the mesh structure of the divider. For mesh, wires having various wire diameters and materials having various opening ratios are commercially available at low costs. In addition, a mesh made of a round wire (a wire having a circular cross-sectional shape) is preferable because the possibility of receiving cell aggregates without cutting becomes high. The mesh is described below, and the same constitution may be achieved using a porous film.

The equivalent-circle-diameter of the opening shape of the through-hole (mesh-hole) of the mesh structure for separation varies depending on the type of cells constituting the cell aggregate to be collected. For example, when the cell is a pluripotent stem cell, an embryonic stem cell, or the like, it is about 5 µm - 150 µm, more preferably 10 µm - 100 µm, further preferably 20 µm - 80 µm. In the following, preferable size and the like of each part are illustrated for cases where the cell is a pluripotent stem cell, an embryonic stem cell or the like, but other cells may also be changed to have appropriate sizes.

The diameter of the wire is about 10 µm - 100 µm, more preferably 30 µm - 80 µm.

With only the above-mentioned definition of equivalent-circle-diameter, an elongated opening shape such as a slit and an intricate opening shape such as a maze are also included, and all small cell aggregates are also trapped. Therefore, in the present invention, in addition to the aforementioned limitation on the equivalent-circle-diameter, the opening shape being a shape capable of accommodating a circle having a diameter of 5 µm to 150 µm (hereinafter referred to as contained circle) is added to the limiting condition. Here, the opening shape being able to accommodate the contained circle also includes the case where the contained circle is inscribed in the opening shape and the case where the contained circle matches the opening shape.

The diameter of the contained circle is more preferably 5 µm - 150 µm, further preferably 10 µm - 100 µm. When the opening shape is circular, the diameter of the contained circle = equivalent-circle-diameter, otherwise, the diameter of the contained circle <equivalent-circle-diameter.

### (Opening shape of mesh-hole)

The opening shape of the mesh-hole in the mesh structure for separation is not particularly limited, and may be triangular, quadrangular, hexagonal, or other polygonal or irregular shape. An opening shape of a general mesh which is economical and has high quality is, for example, a square. In this case, warp and weft wires form an orthogonal lattice mesh as in the example of the mesh structure of the divider shown in Fig. 4(a). Of the four sides of the square, the distance between two parallel sides facing each other may be the diameter of the aforementioned contained circle. It is preferably 5 µm - 150 µm, more preferably, 10 µm - 100 µm.

The mesh structure for separation of the collector is a structure similar to the mesh structure of the divider. The division of the cell aggregate is suppressed by making the wire (beam member) thicker. Even when the thickness of the wire (beam member) is the same as the mesh structure of the divider, the division of the cell aggregate is suppressed by lowering the flow velocity of the liquid medium passing through the mesh structure for separation than the flow velocity of the liquid medium passing through the mesh structure of the divider.

By increasing the volume of the separation chamber where the mesh structure for separation is arranged, making the cross-sectional area of the flow larger than the cross-sectional area of other conduits, or making the total opening area of the mesh-holes in the mesh structure for separation larger than the cross-sectional area of other conduits, the flow velocity of the liquid medium passing through the mesh structure for separation is decreased, and thus cell aggregates are not divided by the mesh structure for separation. As a result, cell aggregates are trapped by the mesh structure for separation and stay in the first chamber 41a. The total opening area of the mesh-holes in the mesh structure for separation is preferably about 0.01-fold to 1000-fold, more preferably about 0.1-fold to 500-fold, the cross-sectional area of other conduits. For example, when the cross-sectional area of other conduits (piping tube) is about 1 mm² - 50 mm², the total opening area of the mesh-holes in the mesh structure for separation is preferably about 0.01 mm² - 50000 mm².

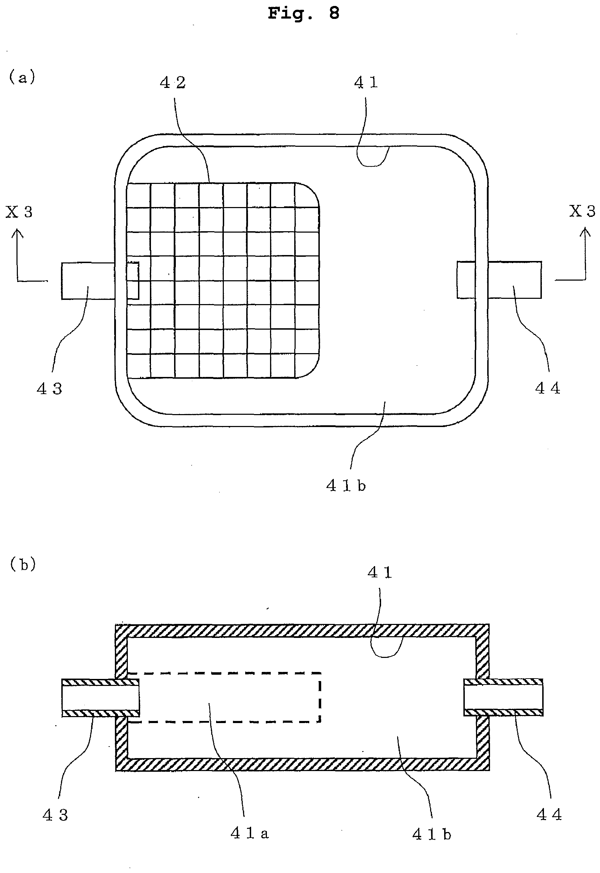

Fig. 8 shows one embodiment of a preferable structure of the collector. In the example of Fig. 8, the overall shape of the mesh structure for separation 42 is bag-like (form of a bag made with a net), and the inner side of the bag-like mesh structure for separation 42 is the first chamber 41a. The separation chamber 41 of the collector is provided with an inlet port 43 and an outlet port 44, and the mesh structure for structure 42 covers the inner opening of the inlet port 43. That is, the inlet port 43 is open to the first chamber 41a which is the inside of the mesh structure for separation 42. On the other hand, the outlet port 44 is open to the second chamber 41b, which is the outside of the mesh structure for separation 42. Contrary to the example of Fig. 8, the liquid medium may be used to flow from the outside to the inside of the mesh structure for separation 42. However, when the mesh structure for separation is flexible and easily deformed, the mesh structure for separation may shrink under the pressure of the flow from the outside to the inside, and the flow of the liquid medium may be obstructed. Therefore, when a bag-like, easily deformable mesh structure for separation is used, the embodiment shown in Fig. 8 in which the liquid medium flows from the inside to the outside of the bag-like mesh structure for separation is preferable. In the case of a bag-like mesh structure for separation which is not easily deformed by the pressure of the flow or imparted with a reinforcing member, the flow direction is not limited. As for the direction of flow of the liquid medium through a bag-like mesh structure for separation (from the inside to the outside, or from the outside to the inside), it is preferable to determine the optimum one in consideration of the number of cell aggregates, flow velocity, viscosity of the medium, and the like.

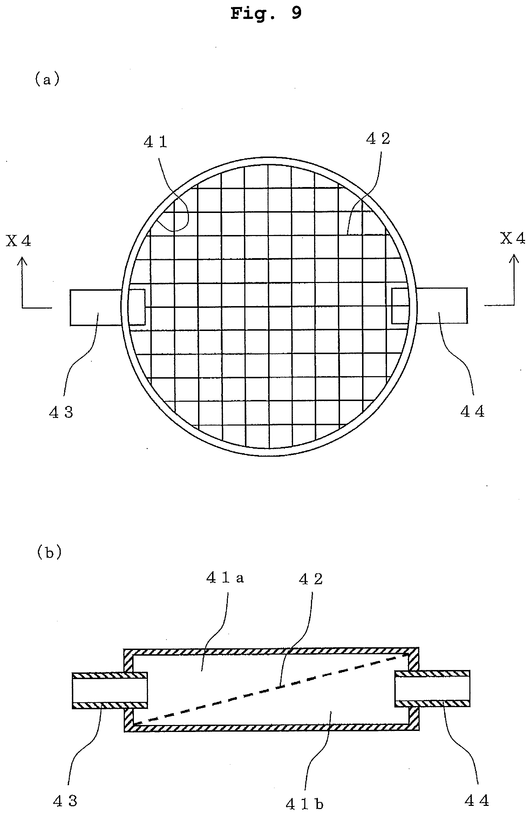

Fig. 9 shows other embodiment of a preferable structure of the collector. In the example of Fig. 9, the separation chamber 41 of the collector is generally a disk (cylindrical), and the entire shape of the mesh structure for separation 42 is flat sheet-like. Similar to the example of Fig. 8, the separation chamber 41 of the collector is provided with an inlet port 43 and an outlet port 44. The mesh structure for separation 42 divides the separation chamber 41 into a first chamber 41a and a second chamber 41b by diagonally crossing the inside of the separation chamber 41 of the collector. With such constitution, the flow of the liquid medium diagonally hits the mesh structure for separation 42 rather than at a right angle. Thus, the effect of preventing clogging is expected.

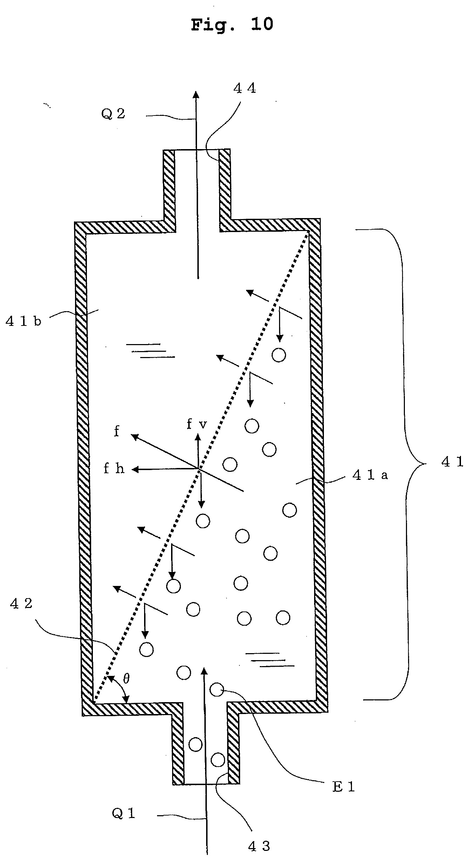

Fig. 10 shows other embodiment of a preferable structure of the collector. In the example of Fig. 10, the entire shape of the mesh structure for separation 42 is flat sheet-like. The shape of the separation chamber 41 of the collector is not particularly limited. Similar to the example of Fig. 8, the separation chamber 41 of the collector is provided with an inlet port 43 from which the liquid medium containing cell aggregates (hereinafter to be also referred to as suspension) Q1, and an outlet port 44 from which the liquid medium Q2 after removal of the cell aggregates goes out. The mesh structure for separation 42 divides the separation chamber 41 into a first chamber 41a and a second chamber 41b by diagonally crossing the inside of the separation chamber 41 of the collector. An important characteristic here is that a mesh structure for separation 42 is provided in the separation chamber 41 such that the advancing direction f of the liquid medium when the liquid medium passes through mesh-holes of the mesh structure for separation 42 has a vertically upward direction component fv. In the example of Fig. 10, the advancing direction f of the liquid medium also has a horizontal direction component fh. In the example of Fig. 10, the mesh structure for separation 42 is a flat sheet-like mesh. The mesh structure for separation is diagonally disposed such that the surface on the second chamber 41b side of the mesh structure for separation becomes an inclined plane, and therefore, the surface on the first chamber 41a side is an overhang surface. The entire surface on the first chamber 41a side of the mesh structure for separation may not be an overhang surface, and the mesh structure for separation may be curved or bent to topically include a surface free from overhanging. The inlet port 43 does not necessarily have to be provided at the bottom of the separation chamber, and the outlet port 44 does not necessarily have to be provided at the top of the separation chamber. In the constitution of Fig. 10, when the inlet port 43 is provided in the upper part of the first chamber 41a and the outlet port 44 is provided in the lower part of the second chamber 41b, the advancing direction f of the liquid medium when the liquid medium passes through the mesh-hole of the mesh structure for separation 42 has a vertically-upward direction component fv even when the inlet port 43 is provided at a position higher than the outlet port 44.

With the above constitution, the cell aggregate E1 trapped on the surface on the first chamber 41a side of the mesh structure for separation 42 is delaminated from the mesh structure for separation and forms sediment. As a result, clogging of the mesh-hole of the mesh structure for separation with the cell aggregate E1 is suppressed, and the liquid medium can efficiently pass through the mesh structure for separation.

### (Placement attitude of mesh structure for separation)

In the example of Fig. 10, the mesh structure for separation is a mesh with a sheet net-like part (i.e., thin plate), the surface (lower surface) on the first chamber 41a side of the mesh structure for separation is an overhang surface and is tilted by an elevation angle θ with respect to the horizontal plane. With the above constitution, the mesh structure for separation diagonally crosses the separation chamber, though depending on the shape of the separation chamber, the area (area of surface with mesh-holes) of the mesh structure for separation tends to become wider, which is preferable because the linear flow velocity can be reduced even when the flow rate is high.

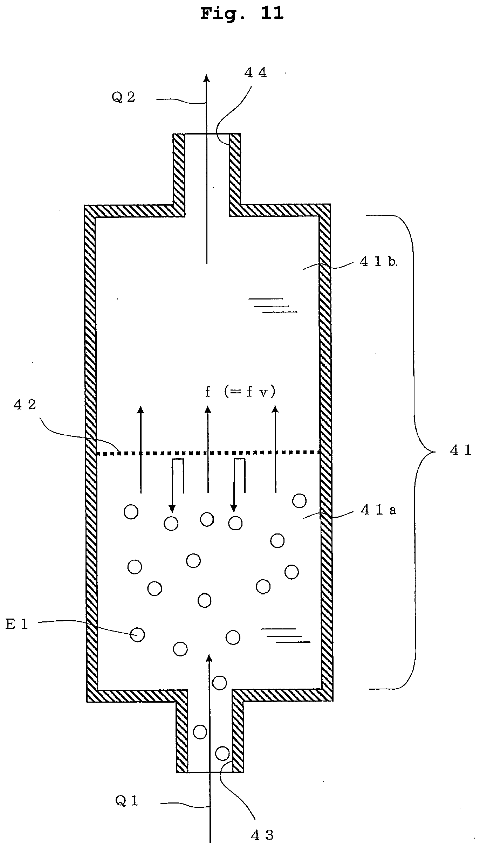

On the other hand, in the example of Fig. 11, similar to the example of Fig. 10, the mesh structure for separation has a mesh with a sheet net-like part. However, the surface (lower surface) on the first chamber 41a side of the mesh structure for separation is a horizontal plane (elevation angle θ of 0) faces directly downward (in other words, the advancing direction f of the liquid medium when the liquid medium passes through the mesh-hole of the mesh structure for separation 42 has a vertically-upward direction component fv alone). Such embodiment is preferable because all of the gravity acting on the cell aggregate trapped in the mesh-hole acts on the cell aggregate as a perpendicular detaching force to the lower surface of the mesh structure for separation, and the clogging can be cleared most effectively.

The angle between the lower surface of the mesh structure for separation and the horizontal plane (elevation angle θ in Fig. 10) may be 0 degrees or more and less than 90 degrees. As in the embodiment of Fig. 10, when the mesh structure for separation is diagonally arranged and the liquid medium is flown into the first chamber from below, a preferable range of the elevation angle θ is 45 degrees ≤ θ < 90 degrees because the effect that the cell aggregate attached to the mesh structure for separation is easily delaminated by the flow of the liquid medium is expected and clogging with cell aggregate is suppressed. As described above, an embodiment in which the lower surface of the mesh structure for separation is a horizontal plane (elevation angle θ = 0 degrees) and an embodiment in which the lower surface of the mesh structure for separation is an oblique overhang surface have own advantages. Therefore, a preferred embodiment may be selected according to the use and the internal shape of the separation chamber.

### (Shape of mesh structure for separation)

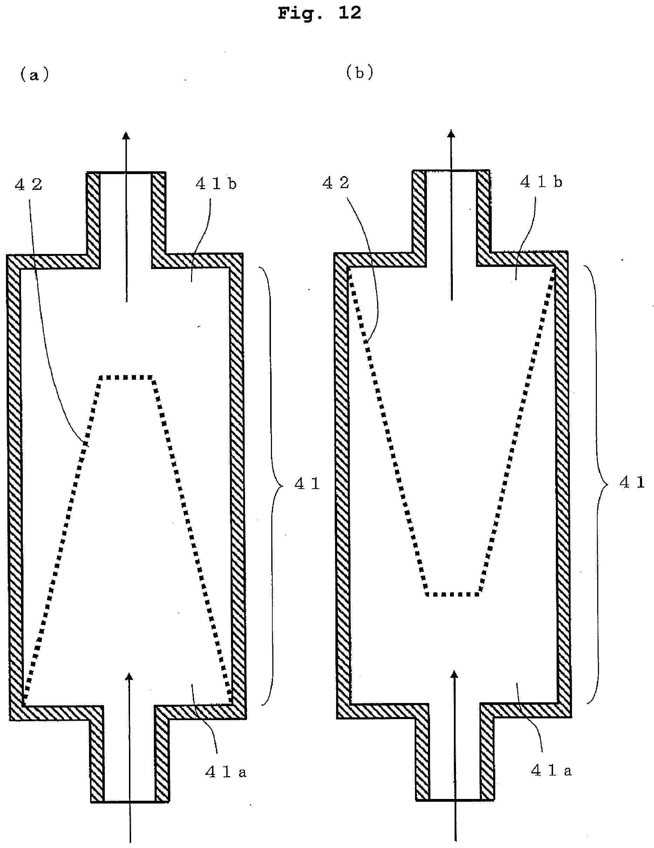

In the embodiment shown in Fig. 12(a), the mesh structure for separation 42 is disposed such that the opening of the bag faces downward, the inside of the bag is a part or all of the first chamber 41a, and the outside of the bag is the second chamber 41b. In the example of Fig. 12(a), the inside of the bag is all of the first chamber 41a. When the position of the bag moves upward in the separation chamber, the inside of the bag becomes a part of the first chamber 41a. The inner surfaces of the wall of the bag, which face each other, are not parallel to each other, and the distance between the inner surfaces becomes narrower from the aforementioned opening toward the deeper inside of the bag. As a result, the inner surface of the bag is entirely an oblique overhang surface or a surface facing directly downward.

In the embodiment shown in Fig. 12(b), the mesh structure for separation 42 is disposed such that the opening of the bag faces upward, the outside of the bag is the first chamber 41a, and the inside of the bag is a part or all of the second chamber 41b. In the example of Fig. 12(b), the inside of the bag is all of the second chamber 41b. When the position of the bag moves downward in the separation chamber, the inside of the bag becomes a part of the second chamber 41b. The inner surfaces of the wall of the bag, which face each other, are not parallel to each other, and the distance between the inner surfaces becomes narrower from the aforementioned opening toward the deeper inside of the bag. As a result, the outer surface of the bag is entirely an oblique overhang surface or a surface facing directly downward.

### (Constitution for disposing mesh structure for separation in separation chamber)

The constitution for disposing the mesh structure for separation in the separation chamber is not particularly limited. For example, a constitution in which the first chamber and the second chamber are prepared as separate parts, and the mesh structure for separation is sandwiched and fixed by these parts, a constitution in which a mesh structure for separation with an adhesive margin is inserted into one separation chamber, the mesh structure for separation is adhered and fixed to the inner wall of the separation chamber using the adhesive margin, and the separation chamber is divided into a first chamber and a second chamber, and the like can be mentioned. Adhesion may be adhesion using an adhesive, welding of parts themselves, or the like.

### (Embodiment of the first chamber having plural inlet/outlet ports)

In the collector, plural ports may be formed in each of the first chamber and the second chamber according to the purpose.

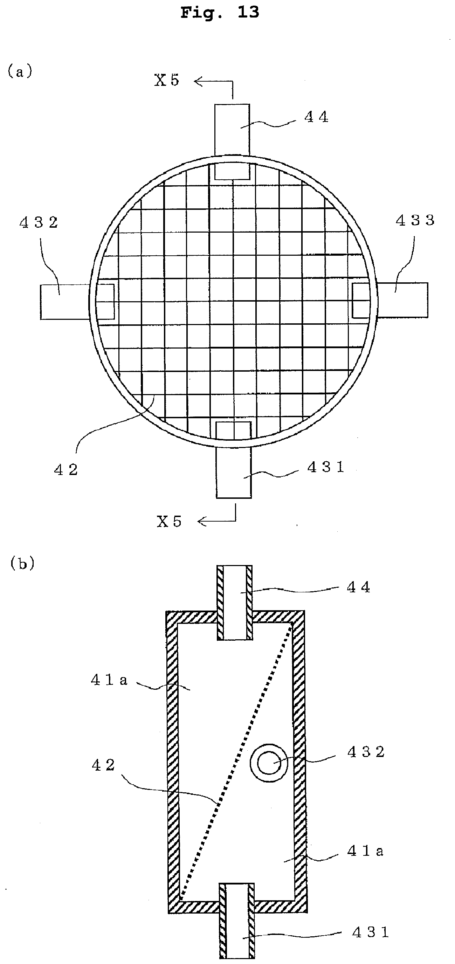

In the embodiment shown in Fig. 13, three ports (first inlet port 431, second inlet port 432, and outlet port 433) are provided in the first chamber, and an outlet port 44 alone is provided in the second chamber. In the embodiment shown in Fig. 13, the outer shape of the separation chamber is disk-like (cylindrical), and the ports 431, 432, 433, and 44 are located at positions with a space of an angle 90 degrees on the outer circumference side surface of the separation chamber.

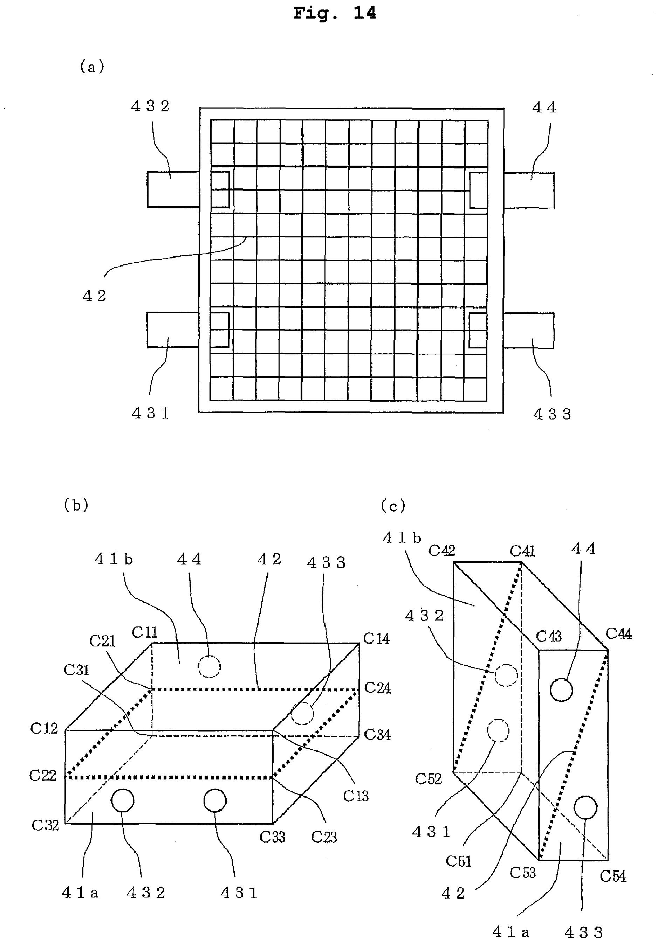

Similarly, in the embodiment shown in Fig. 14, three ports (first inlet port 431, second inlet port 432, and outlet port 433) are provided in the first chamber, and an outlet port 44 alone is provided in the second chamber. In the embodiment shown in Fig. 14, the outer shape of the separation chamber is cuboid-like (thin plate), and as for the ports 431, 432, 433, and 44, two are provided on each of the two surfaces facing each other among the four surfaces on the outer circumference side surface of the separation chamber.

In the embodiment shown in Fig. 14(b), the separation chamber is cuboid-like (thin square plate laid horizontally) with eight vertices (C11, C12, C13, C14, C31, C32, C33, C34). The separation chamber is divided into a first chamber 41a on the lower side and a second chamber 41b on the upper side by a horizontally arranged quadrate sheet-like mesh structure for separation 42. The mesh structure for separation 42 has four vertices C21, C22, C23, C24. The first inlet port 431 and the second inlet port 432 in the first chamber are located in the side surfaces (C22, C23, C33, C32), and the outlet port 433 in the first chamber is located in the side surfaces (C21, C24, C34, C31). In addition, the outlet port 44 of the second chamber is located in the side surfaces (C11, C14, C24, C21).

In the embodiment shown in Fig. 14(c), the separation chamber is cuboid-like (thin square plate stood perpendicular) with eight vertices (C41, C42, C43, C44, C51, C52, C53, C54). The separation chamber is divided into a first chamber 41a on the lower side and a second chamber 41b on the upper side by a diagonally arranged quadrate sheet-like mesh structure for separation 42. The quadrate sheet-like mesh structure for separation 42 has four vertices C41, C52, C53, C44. The first inlet port 431 and the second inlet port 432 in the first chamber are located in the side surfaces (C41, C51, C52), and the outlet port 433 in the first chamber is located in the side surfaces (C21, C24, C34, C31). In addition, the outlet port 44 of the second chamber is located in the side surfaces (C44, C54, C53) .

In both embodiments shown in Fig. 13 and Fig. 14, the first inlet port 431 is a port for allowing the suspension to flow into the first chamber from the outside. The second inlet port 432 is a port for allowing a reagent and/or a liquid for diluting the suspension to flow into the first chamber from the outside. The outlet port 433 provided in the first chamber is a port for flowing a part of the cell aggregate from the first chamber to the outside. These reagents and liquids are not particularly limited as long as they reduce the floating force of the liquid medium and facilitate the separation and/or collection of cell aggregates. Examples of the reagent include, but are not limited to, chelating agents (e.g., EDTA and the like) that steal divalent ions from the structure, thereby decomposing the structure, when a structure composed of a specific polysaccharide such as deacylated gellan gum is contained in the liquid medium. Examples of the liquid for diluting such suspension include, but are not limited to, a liquid medium and a buffer solution and the like that do not contain a fine structure (e.g., deacylated gellan gum, etc.) for suspending a cell aggregate and the like.

With these ports, it is possible to reduce the floating force of the liquid medium and form sediment of the cell aggregate in the collector.

### (Position of inlet port in the first chamber)

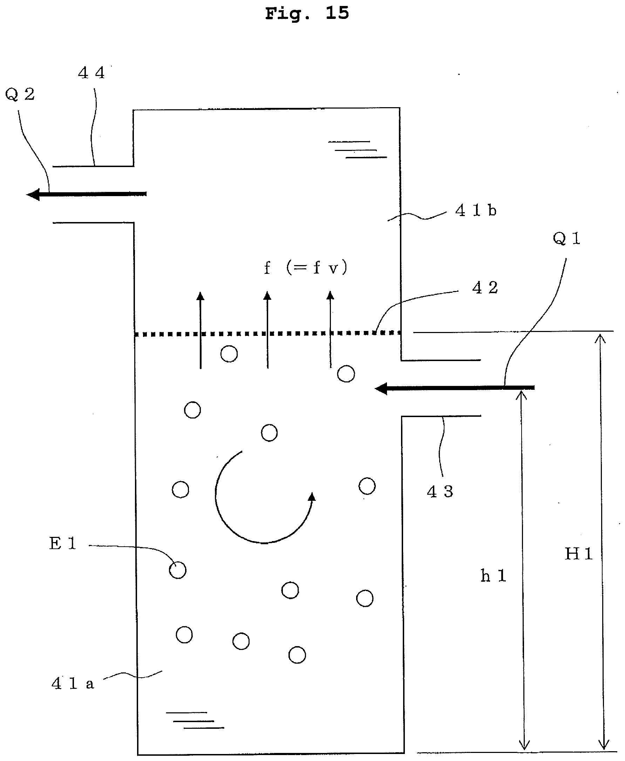

In the embodiment shown in Fig. 15, the second chamber 41b is located above the first chamber 41a, and the sheet-like mesh structure for separation 42 is horizontally arranged. Therefore, the advancing direction f of the liquid when passing through the mesh-holes of the mesh structure for separation 42 is a vertically upward direction.

In the embodiment shown in Fig. 15, the first chamber has at least an inlet a port 43 for the suspension Q1 to flow in from the outside, and the inlet port 43 is provided on the side wall of the first chamber 41a. As a result, the bottom surface of the separation chamber becomes flat, and the collector can be stably arranged on the base surface.

In a preferred embodiment, the height h1 of the central axis of the inlet port 43 is located at a height of 40% or more, preferably 60% or more, of the total height H1 in the room of the first chamber 41a. As a result, a space exists in the first chamber below the height h1 of the central axis of the inlet port. Due to the presence of this space and the flow of the suspension Q1 flowing in from the inlet port 43 provided on the side surface, the cell aggregate preferably circulates in the first chamber 41a, and the cell aggregate is delaminated from the mesh structure for separation and can be accumulated in the space. The cell aggregate accumulated in the space does not interfere with the suspension flowing in newly. Since the inlet port is not provided on the bottom surface of the first chamber, the suspension flowing in newly does not blow up the separation target accumulated at the bottom of the space. Therefore, the liquid medium preferably passes through the mesh structure for separation. The upper limit of the height h1 of the central axis of the inlet port 43 is a position where the upper end of the opening of the inlet port 43 contacts the lower surface of the mesh structure for separation. That is, assuming that the diameter of the opening is d, the upper limit of the height h1 of the central axis of the inlet port 43 is a position lowered by d/2 from the lower surface of the mesh structure for separation.

The position of the outlet port provided in the second chamber is not particularly limited, and it may be provided on the side surface of the second chamber as in the embodiment shown in Fig. 15. In the embodiment shown in Fig. 15, the inlet port provided in the first chamber and the outlet port provided in the second chamber are provided on opposite sides of the side surface of the separation chamber, but they may be provided on the same side surface. The positions of the inlet port and the outlet port are preferably determined appropriately in consideration of the piping work and the piping space occupied by the tube.

### (Example of connecting collector)

As described above, the cell aggregate stays in the first chamber of the collector and is separated from the liquid medium, and only the liquid medium passes through the collector. Fig. 7 also shows a preferable piping connection example for moving the cell aggregate accumulated in the first chamber to the collection container 52 while maintaining the closed system. In the example of this Figure, when the cell aggregate is passed through the collector from the first container 10, switching valves V1 and V2 are operated such that the liquid medium flows through conduit P4, conduit P5, conduit P6, and conduit P7 in this order, and reaches a waste liquid container 50. By this operation, the cell aggregate remains in the first chamber 41a of the collector 40. Then, to take out the cell aggregate staying in the first chamber 41a to the outside, switching valves V1 and V2 are operated such that a new liquid medium flows from the liquid supply container 51 through conduit P7, conduitP6, collector 40, conduit P5, and conduit P8 in this order, and enters collection container 52. By this operation, the new liquid medium passes through the collector 40 in the backward direction (from second chamber 41b to the first chamber 41a), whereby the cell aggregate remaining in the first chamber 41a moves into the collection container 52 and collected without being exposed to the outside air in a preferred embodiment. The liquid supply container 51 may be a syringe. A liquid feeding device may be provided as appropriate. As in the piping example illustrated above, various pipes can be constituted to move the cell aggregate retained in the first chamber 41a with a liquid medium and collect the cell aggregate.

A port for extracting a cell aggregate or a liquid medium for examining the state of the cell aggregate may be appropriately provided in the conduits of the cell culture system. In addition, a sensor (e.g., sensor for measuring lactic acid concentration, glucose concentration, pH, oxygen concentration, carbon dioxide concentration, etc.) for monitoring the state of the liquid medium may be provided while being exposed in the liquid medium in the conduits, or an optical or electrical measuring device may also be provided.

### (Production method of cell aggregate)

The production method of the cell aggregate of the present invention is performed using the above-mentioned cell culture system of the present invention. In the production method, as shown in Fig. 1, a step of dividing a cell aggregate to be divided and a liquid medium by passing the cell aggregate from a supply source through the divider 20 in the cell culture system and a step of feeding the cell aggregate divided in the divider 20 into the first container 10 together with a liquid medium without contacting the outside air in a preferred embodiment, and performing suspension culture in the first container are performed without contact with the outside air in a preferred embodiment. The cell aggregate to be supplied from the supply source S1 is preferably a cell aggregate that has grown to a size to be divided.

### (Culture conditions for cell aggregate)

The conditions for three-dimensionally culturing a cell aggregate by using the cell culture system of the present invention are not particularly limited as long as the cell aggregate grows, and the culture conditions known per se which are suitable for the cells to be cultured can be appropriately used. For example, when using hiPS cells, the temperature condition may be 30°C - 39°C (e.g., 37°C), and the CO₂ concentration may be 1% - 10% (e.g., 5%). The culture period from the division of the first cell aggregate to the division of the second cell aggregate may be 1 to 7 days (e.g., 2 days, 3 days, 4 days, 5 days, 6 days). In one embodiment, cell aggregates can be cultured more efficiently by adding a medium after the start of culturing smaller cell aggregate obtained through the first division. As to the timing of addition of the medium, the cell aggregate can be efficiently cultured by adding a medium in 1 to 5 times, preferably 2 to 4 times, particularly preferably 2 to 3 times, the amount of the culture medium in which the cell aggregates are cultured, on the 1st to 3rd day, preferably 1st to 2nd day, particularly preferably 1st day, from the start of culturing the cell aggregate. In another embodiment, the medium can be added more than once during the culture period from the division of the first cell aggregate to the division of the second cell aggregate. As one example of the embodiment in which the medium is added twice, a medium can be added as the first additional medium in 1 to 5 times (e.g., 2 to 3 times) the amount of the culture medium in which the cell aggregates are cultured at the time of the first day from the start of culturing, and a medium can be added as the second additional medium in 0.1 to 2 times (e.g., 0.4 to 0.7 times) the amount of the culture medium in which the cell aggregates are cultured at the time of the fourth day from the start of culturing. Furthermore, the cell aggregate can also be efficiently cultured by measuring the lactic acid concentration in the medium for culturing the cell aggregate and setting the lactic acid concentration to fall within a specific range. Such lactic acid concentration can be set to generally 0 mM - 20 mM, preferably 0 mM - 18 mM, particularly preferably 0 mM - 16 mM. The lactic acid concentration of the medium may be measured using a sensor or the like provided in the cell culture system of the present invention, as described above.

### (Flow velocity for division)

When the cell aggregate passes through the mesh structure of the divider together with the liquid medium, the flow velocity for preferable division varies depending on the cell type, the size of the cell aggregate, the viscosity of the liquid medium, and the like. It is generally 10 mm/sec - 500 mm/sec, preferably 50 mm/sec - 150 mm/sec.

As the aforementioned flow velocity, the flow velocity at which the liquid (suspension) enters the divider (or the flow velocity out from the divider) can be adopted. The flow velocity can be obtained based on an operation of pushing out or sucking a predetermined amount of solution at a constant speed in a predetermined time using a liquid feed pump such as a syringe and the like. Further, the flow velocity when the liquid passes through the mesh of the mesh structure can be calculated by dividing the amount by the liquid feed pump by the total opening area of the mesh.

### (Backflow washing step to wash divider)

Therefore, in a preferred embodiment of the production method, the above-mentioned backflow washing step for performing the backflow washing of the mesh structure is further added. The backflow washing step is a step of passing the above-mentioned predetermined liquid (a suspension containing a cell aggregate or a cleaning liquid) through the mesh structure in the direction opposite to the direction of passage of the cell aggregate through the mesh structure of the divider for division, thereby washing the mesh structure, after a predetermined amount of cell aggregate has been divided in the step of dividing the cell aggregate.

The frequency of performing the backflow washing step, the constitution of backflow washing of the suspension or flowing a liquid for the washing, the predetermined liquid for washing used in the backflow washing step, flow velocity during washing, and washing time are as described in the explanation of the cell culture system of the present invention.

### (Collection of cell aggregate)

In this production method, as shown in Fig. 7, the cell aggregates divided, suspension cultured, and proliferated in the cell culture system are collected by the collector 40. In the collector, the cell aggregates and the liquid medium are separated by the mesh structure for separation.

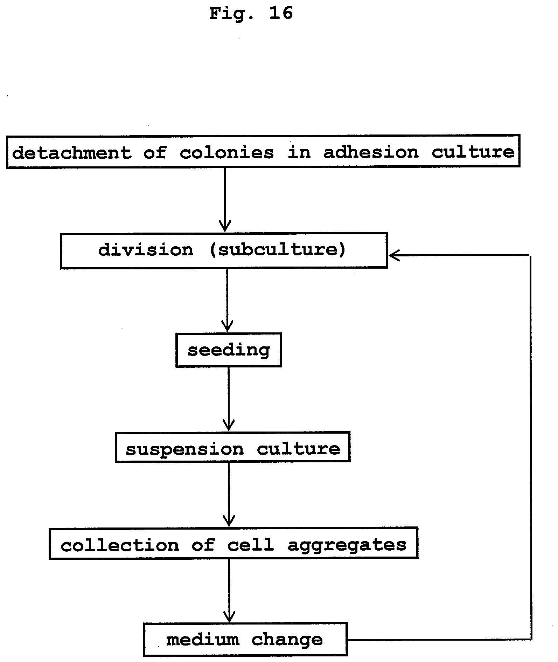

Fig. 16 is a flowchart showing one preferable embodiment of the process of cell culture, division and collection using the cell culture system according to the present invention. The movement of the liquid medium (including cell aggregate) between each step is carried out by piping in a closed system without being exposed to the outside air. As shown in Fig. 1, for example, in supply source S1, cell aggregates obtained by detaching colonies grown by adhesion culture from the scaffold (or cell aggregate formed by suspension culture) are divided by passage through the divider 20. Then, the divided cell aggregates are seeded in a new liquid medium in the first container 10, and suspension cultured to grow the cell aggregates. Then, the grown cell aggregates are collected, the medium is replaced, cell aggregates are returned to the original division step, or fed to the dividing step in the cell culture system of the next stage, and divided by passage through the divider. At this time, instead of delivering all the cell aggregates to the division step, a predetermined ratio of cell aggregates may be taken out as a harvested portion.

### (Flow velocity for collection)

To separate the cell aggregate from the liquid medium by the aforementioned collector, the flow velocity of the liquid medium passing through the collector varies depending on the cell type, the specifications of the mesh structure, the amount of the liquid medium, the viscosity of the liquid medium, and the like. It is preferably 0.01 - 25 mm/sec, more preferably 0.1 - 15 mm/sec, particularly preferably 1 - 10 mm/sec. It is preferable to set the cross-sectional area of the flow path or the flow rate of the liquid feeding device such that the velocity of the liquid medium passing through the individual mesh-holes of the mesh structure for separation is slower than the velocity of the liquid medium passing through the individual mesh-holes of the mesh structure of the divider. In this way, even the same net-like structure can be used for division and collection.

As the aforementioned flow velocity, the flow velocity at which the liquid (suspension) enters the collector (or exits the collector) can be adopted. The flow velocity can be obtained based on the operation of extruding or sucking a predetermined amount of solution at a constant speed in a predetermined time using a liquid feed pump such as a syringe and the like. In addition, the flow velocity when the liquid passes through the mesh of the mesh structure for separation in the collector can be calculated by dividing the flow rate of the aforementioned liquid feed pump by the total opening area of the mesh.

### [Example]

### [Experimental Example 1] Division of cell aggregate of human pluripotent stem cells (hiPS cells)

The hiPS cells were suspension cultured to form a cell aggregate, the cell aggregate was divided using the porous film and a conventional mesh, and a test was conducted to confirm the division performance of the porous film by observing the survival rate of the cell aggregate after each division.

### (Three-dimensional culture of hiPS cell before division)

### Medium 1:

Liquid medium composition prepared by injecting 0.016% (w/v) deacylated gellan gum (KELCOGEL CG-LA, manufactured by Saneigen FFI) using FCeM-series Preparation Kit (manufactured by Nissan Chemical Corporation) to mTeSR-1 medium (manufactured by Stem Cell Technologies) containing 10 mM Y-27632 (manufactured by FUJIFILM Wako Pure Chemical Corporation) according to the mixing method described in patent document 2.

### Medium 2:

Liquid medium composition prepared by injecting 0.016% (w/v) deacylated gellan gum using FCeM-series Preparation Kit to mTeSR1 medium according to the mixing method described in patent document 2.

The hiPS cell line 253G1 (distributed from RIKEN) was cultured in a CO₂ incubator (37°C, 5% CO₂) in a static state using a 15 mL tube, medium 1 and medium 2 (pre-division culture).

On day 0 of pre-division culture, hiPS cell line 253G1 was seeded in medium 1, medium 2 was added every 1 to 2 days, and this was continued for 5 - 6 days to form a cell aggregate. On the final day, the cell aggregate was centrifuged (100×G, 3 min), the supernatant was removed, and the cell aggregate was suspended in medium 1 and then the medium and the cell aggregate were passed through the porous film to divide the cell aggregate. They were seeded in medium 1. (day 0 of culture after division).

### (Specifications of the porous film)

The instruments used in the following cases were all sterilized.

As an example product of the porous film, a porous film of the type shown in Fig. 1 was produced.

The material of the film body is nickel.

As shown in Table 1 below, the thickness of the film body is 20 µm or 40 µm.

The shape of the opening of each through-hole is a regular hexagon congruent with each other, and the through-hole is arranged on the entire film surface of the film body. The pore size of each through-hole (the distance between two parallel sides facing each other among the six sides of the regular hexagon which is the shape of the opening) is 60 µm or 70 µm as shown in Table 1 below.

The wire diameter (width of the beam part) is 20 µm or 40 µm.

The shape of the outer circumference of the film body is circular, and the size (diameter) of the circle is 13 mm.

### (Holder for holding the porous film)

As shown in Fig. 6, a holder for setting the porous film was produced. The inner diameter of the conduit (circular cross section) for flowing the liquid medium composition in which cell aggregates are dispersed is 1.6 mm, and the effective diameter of the flow of the liquid medium composition passing through the porous film is also 1.6 mm.

### (Division using mesh)

As other comparison example of the mesh structure of the divider, a conventional mesh was used.

A nylon mesh (mesh made of nylon wires) having the same outer shape as the above-mentioned porous film and a stainless mesh (mesh made of stainless steel wires) were prepared and mounted on the above-mentioned holder instead of the above-mentioned porous film, and cell aggregates were divided.

The opening shape of the through-hole of the nylon mesh is approximately square, and the length of one side is 70 µm. The diameter of the wire is 50 µm for both the warp and weft wires.

The opening shape of the through-hole of the stainless mesh is approximately square, and the length of one side is 70 µm. The diameter of the wire is 40 µm for both the warp and weft wires.

### (Division of cell aggregate of hiPS cell)

After 5 - 6 days of pre-division suspension culture, cell aggregates were precipitated by centrifugation (100×G, 3 min). The supernatant was removed, and the aggregates were suspended in medium 1 to 2.0×10⁵ cells/mL. 4 mL of the suspension was transferred to a 5 mL syringe (manufactured by Terumo Corporation), and the suspension was passed through the porous film and the mesh at a predetermined passage speed.

### (Culture after division)

The suspension after passing through the porous film and the mesh was seeded in a 15 mL tube and cultured in a CO₂ incubator (37°C, 5% CO₂) in a static state (culture after division). The cap of the 15 mL tube was half-opened. On day 2 of culture after division, 2.5 mL each of medium 2 preheated to 37°C was added. On day 4 of culture after division, 3.5 mL each of medium 2 preheated to 37°C was added.

### (Cell survival rate)

Two hours after the division, the culture tube was removed from the incubator, and the cell aggregates were well dispersed. 0.25 mL of the culture medium was collected and 0.25 mL of ATP reagent (CellTiter-Glo (registered trade mark) Luminescent Cell Viability Assay, manufactured by Promega) was added and the mixture was stirred with Pipetman. After allowing to stand at room temperature for 10 min, 100 µL of each was dispensed into a white 96-well plate, the luminescence intensity (RLU value) was measured with Enspire (manufactured by Perkin Elmer), and the number of viable cells was measured by subtracting the luminescence value of the medium alone.

The relative value when the RLU value (ATP measurement, luminescence intensity) of the suspension before division was 100% was taken as the cell survival rate.

### (Cell proliferation rate)

On day 5 of culture after division, the culture tube was removed from the incubator, and the cell aggregates were well dispersed. 0.5 mL of the culture medium was collected and 0.5 mL of ATP reagent (CellTiter-Glo (registered trade mark) Luminescent Cell Viability Assay, manufactured by Promega) was added and the mixture was stirred with Pipetman. After allowing to stand at room temperature for 10 min, 100 µL of each was dispensed into a white 96-well plate, the luminescence intensity (RLU value) was measured with Enspire (manufactured by Perkin Elmer), and the number of viable cells was measured by subtracting the luminescence value of the medium alone. Taking into consideration the culture volume ratio, a relative value with the RLU value (ATP measurement, luminescence intensity) of the culture medium 2 hrs after the division was taken as the cell proliferation rate.

### (Size and number of cell aggregates after division)

Two hours after the division, the culture tube was removed from the incubator, and the cell aggregates were well dispersed. 1 mL of the culture medium was transferred to a 6-well plate, and the size and number of the cell aggregate were measured with Cell³iMager (manufactured by SCREEN Holdings). The culture medium used for the measurement was not returned to the tube. From the measurement results, the equivalent-circle-diameter, the number of cell aggregates, and the proportion of cell aggregates having an equivalent-circle-diameter of 120 µm or more were calculated.

The above test results are shown in the following Table 1.

**[Table 1]**

| Case | form of mesh | pore size (µm) | wire diameter (µm) | passage speed (cm/sec) | cell survival rate (%) | cell proliferation rate | equivalent -circle-diameter (µm) | number | proportion of cell aggregates with diameter of 120 µm or more |
|---|---|---|---|---|---|---|---|---|---|
| (before crushing) | - | - | - | - | 100 | - | 149.0 | 214 | - |
| comparison example 1 | nylon mesh | 70 | 50 | 15 | 97 | 3.73 | 95.9 | 535 | 22.1% |
| comparison example 2 | stainless mesh | 70 | 40 | 15 | 87 | 4.13 | 92.9 | 542 | 17.0% |
| comparison example 3 | nylon mesh | 70 | 50 | 7.5 | 74 | 3.82 | 105.1 | 260 | 28.8% |
| comparison example 4 | stainless mesh | 70 | 40 | 7.5 | 76 | 3.23 | 96.7 | 322 | 19.6% |
| Example 1 | porous film | 70 | 40 | 15 | 75 | 3.78 | 88.4 | 522 | 14.4% |
| Example 2 | porous film | 70 | 20 | 15 | 71 | 4.01 | 90.1 | 520 | 14.7% |
| Example 3 | porous film | 60 | 20 | 15 | 77 | 3.98 | 80.4 | 654 | 6.9% |
| Example 4 | porous film | 70 | 20 | 7.5 | 86 | 4.49 | 91.4 | 461 | 14.1% |
| Example 5 | porous film | 60 | 20 | 7.5 | 76 | 3.66 | 83.6 | 605 | 7.6% |

As is clear from Table 1 above, it was clarified that, in the processing speed region of not more than 15 cm/sec, compared with the mesh, the porous film could divide into smaller cell aggregates and the number of cell aggregates after the division was larger.

Also, it was clarified that the porous film can reduce the proportion of cell aggregates of 120 µm or more and can divide into cell aggregates with more uniform size than the mesh.

### [Experimental Example 2] Culture of human pluripotent stem cell (hiPS cell)

### (adhesion maintenance culture)

hiPS cell line 253G1 (distributed by RIKEN) cells were seeded in a 6 cm culture dish coated with vitronectin VTN-N (manufactured by ThermoFisher Scientific, #A14700) at 5.0×10⁴ cells - 5.0×10⁵ cells, and cultured using mTeSR1 (registered trade mark) medium (manufactured by STEMCELL Technologies, #ST-05850). After seeding, the cells were cultured in mTeSR1 medium containing 10 µM Y-27632 (manufactured by FUJIFILM Wako Pure Chemical Industries, Ltd., #030-24021) for 24 hr, and thereafter the medium was replaced every day with mTeSR1 medium not containing 10 µM Y-27632. 3 - 4 days after seeding, the cells were detached using PBS solution containing 0.5 mM EDTA, whereby subculture was maintained.

### (Suspension maintenance culture)

Medium 1: Composition prepared using 0.016% (w/v) deacylated gellan gum (KELCOGEL CG-LA, manufactured by Saneigen FFI) and FCeM-series Preparation Kit (manufactured by Nissan Chemical Corporation) to mTeSR1 medium containing 10 µM Y-27632 according to the method of patent document 1 (WO 2016/163444)

Medium 2: Composition prepared using 0.016% (w/v) deacylated gellan gum and FCeM-series Preparation Kit to mTeSR1 medium according to the method of patent document 1

To carry out three-dimensional culture, the hiPS cell line 253G1 used for seeding was detached as a colony state using Dissociation Solution for human ES/iPS Cells (manufactured by REPROCELL, #RCHETP002). After suspending the detached cells in medium 1, the cells were divided using a porous film (opening shape is regular hexagon, pore size (distance between two parallel sides) 60 µm, wire diameter (beam width) 20 µm, passage speed 5 cm/sec) to produce cell aggregates. They were seeded at a cell concentration of 1.0×10⁵ cells/mL - 2.0×10⁶ cells/mL, and three-dimensionally cultured in a CO₂ incubator (37°C, 5%CO₂) in a stationary state. On day 0 of culture, they were seeded in medium 1, medium 2 was added every 1 to 2 days, and the culture was continued for 5 to 7 days. The formed cell aggregates were collected using a 30 µm mesh (manufactured by Miltenyi Biotec, #130-110-915), suspended in medium 1, and divided using a porous film (opening shape of regular hexagon, pore size (distance between parallel two sides) 60 µm, wire diameter (beam width) 20 µm, pass through speed 5 cm/sec), whereby subculture was maintained.

### [Experimental Example 3] Study of three-dimensional culture conditions of human pluripotent stem cell (hiPS cell)

The cell aggregates of hiPS cell line 253G1 produced by the method of Experimental Example 2, and cultured by three-dimensional culture for not less than one passage were counted on day 5, the cell concentration was adjusted to 0.3×10⁶ cells/mL or 0.6×10⁶ cells/mL or 1.2×10⁶ cells/mL. The cells were divided using a porous film (opening shape is regular hexagon, pore size (distance between two parallel sides) 60 µm, wire diameter (beam width) 20 µm, passage speed 5 cm/sec), seeded in a 15 mL tube (manufactured by Falcon, model number 352095), and three-dimensionally cultured in a CO₂ incubator (37°C, 5%CO₂) in a stationary state without sealing with a lid. On day 0 of culture, the cells were seeded in medium 1, medium 2 was added after day 1 under various conditions, and the cells were cultured for 4 - 6 days. In addition, the condition in which the same operation was performed without culturing the cells was used as a negative control.

condition 1: no addition of medium
condition 2: equal amount of fresh medium 2 was added on day 1 to cell culture medium
condition 3: equal amount of fresh medium 2 was added on day 2 to cell culture medium
condition 4: equal amount of fresh medium 2 was added on days 1 and 3 to cell culture medium
condition 5: 2-fold amount of fresh medium 2 was added on day 1 to cell culture medium
condition 6: 3-fold amount of fresh medium 2 was added on day 1 to cell culture medium

### (Size profile of cell aggregate)

The culture tube was removed from the incubator, and the cell aggregates were well dispersed. A part of the culture medium was placed in a 12 well plate, diluted with DMEM/Ham's F-12 (manufactured by FUJIFILM Wako Pure Chemical Corporation, #048-29785), the size of the cell aggregates with a diameter of 50 µm or more was measured, and the average diameter was calculated by Cell³iMagerduos (manufactured by SCREEN Holdings).

In the measurement of the size of the cell aggregates, the equivalent-circle-diameter of the cell aggregate was measured, and the mean thereof was taken as the average diameter. The average diameter of the cell aggregate of less than 100 µm is marked with Δ, not less than 100 µm and less than 120 µm is marked with ○, not less than 120 µm is marked with ⊙. The condition without the test is marked with -.

**[Table 2]**

| | concentration of seeded cells (×10^6 cells/mL) | day 1 | day 2 | day 3 | day 4 | day 5 | day 6 |
|---|---|---|---|---|---|---|---|
| condition 1 | 0.3 | Δ | Δ | ○ | ○ | - | - |
| | 0.6 | Δ | Δ | Δ | ○ | - | - |
| | 1.2 | Δ | Δ | Δ | Δ | - | - |
| condition 2 | 0.3 | Δ | Δ | ○ | ⊙ | ⊙ | ⊙ |
| | 0.6 | Δ | Δ | ○ | ○ | Δ | ○ |
| | 1.2 | Δ | Δ | Δ | Δ | Δ | Δ |
| condition 3 | 0.3 | Δ | Δ | ○ | ○ | - | - |
| | 0.6 | Δ | Δ | Δ | ○ | - | - |
| | 1.2 | Δ | Δ | Δ | Δ | - | - |
| condition 4 | 0.3 | Δ | Δ | ○ | ⊙ | ○ | ⊙ |
| | 0.6 | Δ | Δ | ○ | ○ | ○ | ○ |
| | 1.2 | Δ | Δ | Δ | Δ | Δ | Δ |
| condition 5 | 0.3 | Δ | ○ | ○ | ⊙ | - | - |
| | 0.6 | Δ | ○ | ⊙ | ⊙ | - | - |
| | 1.2 | Δ | ○ | ○ | ○ | - | - |
| condition 6 | 0.3 | Δ | ○ | ○ | ⊙ | - | - |
| | 0.6 | Δ | ○ | ○ | ⊙ | - | - |
| | 1.2 | Δ | ○ | ○ | ⊙ | - | - |

### (Medium environment measurement)

The culture tube was removed from the incubator, and the cell aggregates were well dispersed. 0.1 mL of the culture medium was collected and lactic acid concentration (mM) of the medium was measured using Vi-CELL MetaFLEX (manufactured by Beckman Coulter). Lactic acid is a by-product of cell metabolism and can be toxic to cells. Therefore, it is possible to determine the optimum amount of the medium to be added and the timing of subculture by monitoring the concentration of lactic acid in the culture medium. In addition, the conditions under which the test was not conducted are indicated with -.

**[Table 3]**

| | concentration of seeded cells (×10^6 cells/mL) | day 0 | day 1 | day 2 | day 3 | day 4 | day 5 | day 6 |
|---|---|---|---|---|---|---|---|---|
| condition 1 | negative control | 0.0 | 0.0 | 0.0 | 0.0 | 0.0 | - | - |
| | 0.3 | 0.0 | 1.8 | 5.2 | 9.0 | 13.4 | - | - |
| | 0.6 | 0.0 | 3.6 | 9.1 | 12.5 | 15.3 | - | - |
| | 1.2 | 0.0 | 6.7 | 12.1 | 13.8 | 15.7 | - | - |
| condition 2 | negative control | 0.0 | 0.0 | 0.0 | 0.0 | 0.0 | 0.0 | 0.1 |
| | 0.3 | 0.0 | 1.8 | 2.8 | 5.4 | 9.0 | 10.9 | 13.0 |
| | 0.6 | 0.0 | 3.6 | 5.4 | 9.2 | 13.1 | 13.0 | 14.9 |
| | 1.2 | 0.0 | 6.7 | 8.4 | 11.9 | 14.5 | 13.9 | 14.3 |
| condition 3 | negative control | 0.0 | 0.0 | 0.0 | 0.1 | 0.0 | - | - |
| | 0.3 | 0.0 | 1.8 | 5.2 | 5.1 | 8.2 | - | - |
| | 0.6 | 0.0 | 3.6 | 9.1 | 8.4 | 11.7 | - | - |
| | 1.2 | 0.0 | 6.7 | 12.1 | 7.8 | 10.3 | - | - |
| condition 4 | negative control | 0.0 | 0.0 | 0.0 | 0.0 | 0.0 | 0.0 | 0.1 |
| | 0.3 | 0.0 | 1.8 | 2.8 | 5.4 | 4.8 | 7.9 | 14.0 |
| | 0.6 | 0.0 | 3.6 | 5.4 | 9.2 | 7.2 | 10.1 | 13.0 |
| | 1.2 | 0.0 | 6.7 | 8.4 | 11.9 | 8.7 | 11.2 | 13.0 |
| condition 5 | negative control | 0.0 | 0.0 | 0.0 | 0.0 | 0.0 | - | - |
| | 0.3 | 0.0 | 1.8 | 2.0 | 3.8 | 6.5 | - | - |
| | 0.6 | 0.0 | 3.6 | 3.9 | 7.2 | 12.4 | - | - |
| | 1.2 | 0.0 | 6.7 | 5.7 | 9.3 | 13.2 | - | - |
| condition 6 | negative control | 0.0 | 0.0 | 0.0 | 0.0 | 0.0 | - | - |
| | 0.3 | 0.0 | 1.8 | 1.6 | 2.9 | 5.7 | - | - |
| | 0.6 | 0.0 | 3.6 | 2.9 | 5.3 | 9.5 | - | - |
| | 1.2 | 0.0 | 6.7 | 4.3 | 7.5 | 13.0 | - | - |

As a result of the test, the cell aggregates did not grow to 120 µm or more in 4 days under the conditions where no medium is added after seeding and the conditions where the same amount of medium as the culture medium is added on day 2. On the other hand, under conditions where the same amount of medium as the culture medium is added on day 1 and days 1 and 3 of culture, the cell aggregates grew to 120 µm or more in 4 days only at a cell concentration of 0.3×10⁶ cells/mL. Furthermore, under conditions where 3 times the amount of the culture medium is added on day 1, the cell aggregates grew to 120 µm or more in 4 days only at any cell concentration. From the above results, it was clarified that the cell aggregates can be efficiently cultured at any cell concentration by adding 3 times the amount of the medium to the culture solution on day 1 of culture. From the medium environment measurement results, it was suggested that culture can be performed at a lactic acid concentration of 0 - 15.7 mM, and can be efficiently performed at 0 - 14.0 mM.

### [Experimental Example 4] Study of collection conditions of human pluripotent stem cell (hiPS cell) aggregate

A cell culture medium of hiPS cell line 253G1 on day 7 that was cultured for 1 passage or more by three-dimensional culture according to the method of Experimental Example 2 was used. As a mesh structure for separation, nylon meshes with wire diameter 50 µm, pore size (length of one side of square) 15 µm, 36 µm, 50 µm, 70 µm were set on a filter holder (manufactured by Merck, #SX0001300, effective area 0.7 cm²), and the cell culture medium was flown at various flow velocities, whereby cell aggregates were trapped on the mesh.

Here, the effective area is the area of the mesh that contributes to the separation of the cell aggregate, that is, the area of the region where the culture medium can actually pass through and the cell aggregate can be trapped (total of the opening area and the area occupied by wires).

### (Measurement of number and average diameter of cell aggregates)

The cell aggregates were well dispersed. A part of the culture medium used in the test was placed in a 12 well plate, and the number and size of the cell aggregates with a diameter of 50 µm or more in the culture medium was measured. In the measurement of the size of the cell aggregates, the equivalent-circle-diameter of the cell aggregate was measured, and the mean thereof was taken as the average diameter. As a result, the concentration of the cell aggregate was 700 aggregates/mL and the average diameter was 145.8 µm.

### (Calculation of cells that passed through mesh)

The culture medium, and 0.5 mL of the filtrate were collected, 0.5 mL of ATP reagent (CellTiter-Glo (registered trade mark) Luminescent Cell Viability Assay, manufactured by Promega) was added to each and the mixture was stirred with Pipetman. After allowing to stand at room temperature for 10 min, 200 µL of each was dispensed into a white 96-well plate, the luminescence intensity (RLU value) was measured with Enspire (manufactured by Perkin Elmer), and the number of viable cells in the filtrate that passed through the mesh was measured by subtracting the luminescence value of the medium alone. The relative value of the filtrate when the RLU value (ATP measurement, luminescence intensity) of the culture medium was 100% was taken as the mesh subcultured cell. The test results are shown in Table 4.

**[Table 4]**

| pore size (µm) | passage speed (cm/sec) | cells that passed mesh (%) |
|---|---|---|
| 15 | 0.1 | 1.7 |
| 36 | 0.1 | 0.9 |
| 50 | 0.1 | 1.0 |
| 70 | 0.1 | 8.6 |

As shown in Table 4, it was clarified that, when cell aggregates are collected at a pass through speed of 0.1 cm/sec, 8-fold or more cells flow out into the filtrate when using a mesh with a pore size (length of one side of square) of 70 µm as compared to meshes with pore sizes of 36 µm and 50 µm. On the other hand, it was clarified that, when a mesh with pore size 15 µm was used, about 1.7- to 1.9-fold cells flow out into the filtrate as compared to meshes with pore sizes of 36 µm and 50 µm. From the above results, it was suggested that, when cell aggregates are collected at a pass through speed of 0.1 cm/sec, cells can be efficiently collected by using meshes with pore sizes of 36 µm and 50 µm.

Using a mesh with wire diameter 50 µm, pore size (length of one side of square) 50 µm, and changing the pass through speed, the proportion of the cell aggregates that passed through the mesh was investigated. The test results are shown in Table 5.

**[Table 5]**

| pore size (µm) | passage speed (cm/sec) | cells that passed mesh (%) |
|---|---|---|
| 50 | 0.1 | 1.0 |
| 50 | 0.7 | 2.1 |
| 50 | 1.4 | 3.7 |
| 50 | 7.1 | 7.9 |

As shown in Table 5, it was suggested that, when cell aggregates are collected using a mesh with a pore size of 50 µm, the cell aggregates can be efficiently collected at a pass through speed of 0.1 cm/sec.

Using a mesh with wire diameter 50 µm, pore size (length of one side of square) 50 µm, setting the pass through speed to 0.1 cm/sec, and changing the number of cell aggregates ((total cell aggregate number)/(filter effective area)) per unit area, the proportion of the cell aggregates that passed through the mesh was investigated. The test results are shown in Table 6.

**[Table 6]**

| pore size (µm) | passage speed (cm/sec) | number of treated cell aggregates (cell aggregates) | number of cell aggregates per uni t area (cell aggregates/cm²) | cells that passed mesh |
|---|---|---|---|---|
| 50 | 0.1 | 2100 | 3000 | 1.0 |
| 50 | 0.1 | 4200 | 6000 | 2.6 |
| 50 | 0.1 | 8400 | 12000 | 42.8 |

As shown in Table 6, it was clarified that, when cell aggregates are collected using a mesh with a pore size of 50 µm at a pass through speed of 0.1 cm/sec and 12000 cell aggregates are collected per unit area, about 40% of the cells flow out into the filtrate. On the other hand, it was suggested that cell aggregates can be efficiently collected when they are 6000 or less per unit area.

### [Experimental Example 5] Collection of cell aggregates of human pluripotent stem cell (hiPS cell)

A cell culture medium of hiPS cell line 253G1 on day 5 - 6 that was cultured for 1 passage or more by three-dimensional culture according to the method of Experimental Example 2 was passed through a mesh container for collection under various conditions different in the mesh pore size, effective area, and shape at a speed of 1 mL/sec to trap the cell aggregates in the mesh. Thereafter, the cell aggregates were collected by backflow of the fresh medium 2 described in Experimental Example 2.

### (Specifications of collector)

Under conditions 7 - 9, a collector in which the bag-like mesh shown in Fig. 8 was disposed was used. In condition 7, a mesh having a hole diameter of 21 µm and an effective area of about 1.5 cm² was used.

The effective area of the collector is the area of the mesh that contributes to the collection of the cell aggregate, that is, the area of the region where the culture medium can actually pass through and the cell aggregate can be trapped (total of the opening area and the area occupied by wires).

Under condition 8, a mesh having a mesh pore size of 40 µm and an effective area of about 1.5 cm² was used. Under condition 9, a mesh having a mesh pore size of 30 µm and an effective area of about 16 cm² was used. The trapped cell aggregates were moved to the collection container by reversing the flow direction at the time of collection with respect to the flow direction at the time of trapping.

Under condition 10, a collector in which the disk-shaped mesh shown in Fig. 9 was arranged was used. The pore size of the mesh was 70 µm and the effective area was about 12 cm². The trapped cell aggregates were moved to the collection container by reversing the flow direction at the time of collection with respect to the flow direction at the time of trapping.

### (Measurement of number and average diameter of cell aggregates)

The cell aggregates were well dispersed. A part of the culture medium used in the test was placed in a 12 well plate, and the number and size of the cell aggregates with a diameter of 50 µm or more in the culture medium were measured. In the measurement of the size of the cell aggregates, the equivalent-circle-diameter of the cell aggregate was measured, and the mean thereof was taken as the average diameter.

### (Calculation of collection rate)

The culture medium, and 0.5 mL of the collection solution were collected, 0.5 mL of ATP reagent (CellTiter-Glo (registered trade mark) Luminescent Cell Viability Assay, manufactured by Promega) was added to each and the mixture was stirred with Pipetman. After allowing to stand at room temperature for 10 min, 200 µL of each was dispensed into a white 96-well plate, the luminescence intensity (RLU value) was measured with Enspire (manufactured by Perkin Elmer), and the number of viable cells in the filtrate that passed through the mesh was measured by subtracting the luminescence value of the medium alone. The relative value of the collection solution with the RLU value (ATP measurement, luminescence intensity) of the culture medium as 100% was taken as the collection rate.

The results of the collection rate are shown in Table 7.

**[Table 7]**

| | condition 7 | condition 8 | condition 9 | condition 10 |
|---|---|---|---|---|
| shape of collector | Fig. 8 | Fig. 8 | Fig. 8 | Fig. 9 |
| effective area (cm²) | 1.5 | 1.5 | 16 | 12 |
| mesh pore size (µm) | 21 | 40 | 30 | 70 |
| speed (mL/sec) | 1 | 1 | 1 | 1 |
| passage speed (cm/sec) | 0.67 | 0.67 | 0.06 | 0.08 |
| number of cell aggregates (cell aggregates/mL) | 1028 | 1028 | 384 | 595 |
| total number of cell aggregates (cell aggregates) | 1028 | 1028 | 19200 | 17850 |
| number of cell aggregates per unit area (cell aggregates / cm²) | 685 | 685 | 1200 | 1488 |
| cell aggregate average diameter (µm) | 187.4 | 187.4 | 128.8 | 126.2 |
| collection rate (%) | 60.5 | 44.7 | 48.1 | 73.6 |

As shown in Table 7, it was clarified that a smaller mesh pore size permits more efficient collection of cell aggregates when the cell aggregates are collected using the collector having the shape shown in Fig. 8 and the effective area is the same as shown in condition 7 and condition 8. On the other hand, it was clarified that, when the cell aggregates are collected using the collector having the shape shown in Fig. 9, they can be efficiently collected under condition 10 even though the mesh pore size is larger than that under condition 9. From the above results, it was suggested that the mesh pore size and the shape of the collector need to be devised in order to efficiently collect the cells.

### [Experimental Example 6] Confirmation of the effect of backflow washing of mesh structure

In this Experimental Example, the effect of backflow washing on the mesh structure was investigated by repeating the following operations (i) - (iii).
(i) Cell aggregates are divided by the mesh structure of the divider, with a sample solution containing cell aggregates having a predetermined density and a predetermined flow velocity.
(ii) Every time a predetermined amount of cell aggregates passes, the survival rate of the cell aggregates that have passed through is measured.
(iii) Every time a predetermined amount of the cell aggregates has passed through in the aforementioned (ii), backflow washing is performed on the mesh structure.

The cell aggregate used in the test is a cell aggregate composed of human pluripotent stem cells (hiPS cells).

### (Three-dimensional culture of hiPS cell before division)

### Medium 1:

Liquid medium composition prepared by injecting 0.016% (w/v) deacylated gellan gum (KELCOGEL CG-LA, manufactured by Saneigen FFI) using FCeM-series Preparation Kit (manufactured by Nissan Chemical Corporation) to mTeSR1 medium (manufactured by TEMCELL Technologies) containing 10 µM Y-27632 (manufactured by FUJIFILM Wako Pure Chemical Corporation) according to the mixing method described in patent document 2.

### Medium 2:

Liquid medium composition prepared by injecting 0.016% (w/v) deacylated gellan gum to mTeSR1 medium according to the mixing method described in patent document 2.

The hiPS cell line 253G1 (distributed from RIKEN) was maintenance cultured in a CO₂ incubator (37°C, 5% CO₂) in a static state using a variable volume 200 mL culture bag (manufactured by Nipro), medium 1 and medium 2.

The cells were seeded in medium 1 on day 0 of culture, medium 2 was added every 1 to 3 days, and this was continued for 6 to 8 days to form a cell aggregate.

On the final day, the cell aggregate was collected using MACS (registered trade mark) Smart Stratiners (70 µm, manufactured by MACS), suspended in medium 1, and then passed through a device according to the device of the present invention, and the divided cell aggregates were seeded (day 0).

This was repeated to carry out maintenance culture of the cells.

### (Specifications of the divider)

The instruments of each part used in this test were electron beam sterilized.

As an example product of the mesh structure, a porous film of the type shown in Fig. 3 was produced.

The material of the porous film is nickel, the thickness of the porous film is 20 µm, and the width of the beam part is 20 µm. The pore size of each through-hole (the distance between two parallel sides facing each other among the six sides of the regular hexagon which is the shape of the opening) is 70 µm.

The matters except for those specified are the same as in the porous film used in Experimental Example 1.

The shape of the outer circumference of the porous film is circular, and the diameter of the circle is 6 mm.

### (Holder for holding mesh structure)

As shown in Fig. 6, a holder for setting the mesh structure was produced. A divider can be provided by setting the mesh structure in the holder. The inner diameter of the conduit (circular cross section) for flowing the liquid medium composition in which cell aggregates are dispersed is 2.6 mm, and the effective diameter of the flow of the liquid medium composition passing through the device is also 2.6 mm.

### (Division of cell aggregate of hiPS cells)

After suspension culture for 7 days, the cell aggregates were collected using MACS (registered trade mark) Smart Stratiners (70 µm, manufactured by MACS), and suspended in medium 1 to produce two kinds of suspensions having different concentrations (3.0×10⁵ cells/mL and 6.0×10⁵ cells/mL).
(i) Experimental Example in which division was continued without backflow washing of the mesh structure.
   50 mL each of the aforementioned two kinds of suspensions was transferred to two 50 mL syringes (manufactured by Nipro), each suspension was passed through the above-mentioned divider at a velocity of 10 cm/sec, dispensed into a 15 mL tube each time 10 mL of the suspension passed through the divider, and 5 tubes containing the sample after division of the suspension at a concentration of 3.0×10⁵ cells/mL and 5 tubes containing the sample after division of the suspension at a concentration of 6.0×10⁵ cells/mL were obtained.
(ii) Experimental Example in which division was continued while performing periodic backflow washing of mesh structure.

An operation was repeated in which 50 mL each of the aforementioned two kinds of suspensions was transferred to two 50 mL syringes (manufactured by Nipro), each suspension was passed through the above-mentioned divider at a velocity of 10 cm/sec, dispensed into a 15 mL tube each time 10 mL of the suspension passed through the divider, and the syringe was operated to allow 1 mL of the suspension to flow backward at a flow velocity of 100 mm/sec to perform backflow washing of the mesh structure after distribution to the tube, after which 10 mL suspension was re-flown, divided, and distributed to another 15 mL tube. As a result, similar to the aforementioned (i), 5 tubes containing the sample after division of the suspension at a concentration of 3.0×10⁵ cells/mL and 5 tubes containing the sample after division of the suspension at a concentration of 6.0×10⁵ cells/mL were obtained.

The 15 mL tubes (4 kinds, 20 tubes in total) after distribution were allowed to stand in an incubator (37°C, 5% CO₂) for 2 hrs.

### (Measurement of cell survival rate)

After standing for 2 hours, the aforementioned 15 mL tube was removed from the incubator, the cell aggregates were well dispersed by blending with inversion. 0.75 mL of the culture medium was collected and 0.75 mL of ATP reagent (CellTiter-Glo (registered trade mark) Luminescent Cell Viability Assay, manufactured by Promega) was added and the mixture was well stirred with Pipetman. After allowing to stand at room temperature for 10 min, 100 µL of each was dispensed into a white 96-well plate, the luminescence intensity (RLU value) was measured with Enspire (manufactured by Perkin Elmer), and the number of viable cells was measured by subtracting the luminescence value of the medium alone.

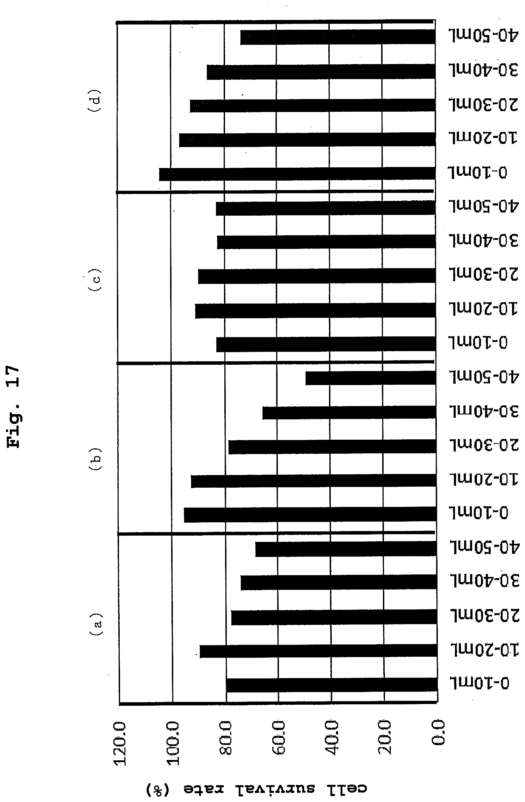

The relative value when the RLU value (ATP measurement, luminescence intensity) of the suspension before division was 100% was taken as the cell survival rate. The cell survival rate is shown in the graph of Fig. 17.

### (Evaluation of effect of backflow washing of mesh structure)

The graphs of Figs. 17(a) and (b) show the results of Experimental Example in which the division was continued without performing the backflow washing of the mesh structure of the above-mentioned (i). The graph of Fig. 17(a) shows the results relating to a suspension having a cell concentration of 3.0×10⁵ cells/mL, and the graph of Fig. 17(b) shows the results relating to a suspension having a cell concentration of 6.0×10⁵ cells/mL.

The graphs of Figs. 17(c) and (d) show the results of Experimental Example in which the division was continued while performing the backflow washing of the mesh structure of the above-mentioned (ii). The graph of Fig. 17(c) shows the results relating to a suspension having a cell concentration of 3.0×10⁵ cells/mL, and the graph of Fig. 17(d) shows the results relating to a suspension having a cell concentration of 6.0×10⁵ cells/mL.

As shown in the graphs of Figs. 17(a) and (b), when the backflow washing of the mesh structure was not performed, cell survival rate decreased as the amount of the dividing treatment increased at both cell densities. In contrast, as shown in the graphs of Figs. 17(c) and (d), when the backflow washing of the mesh structure was performed, a decrease in the cell survival rate was sufficiently suppressed at a cell density of 3×10⁵ cells/mL, and a decrease in the cell survival rate was suppressed even at a cell density of 6×10⁵ cells/mL as compared to the results shown in the graph of Fig. 17(b).

From the above, it was clarified that in the division of the cell aggregate using the mesh structure, the periodic backflow washing of the mesh structure is very effective in suppressing a decrease in the survival rate of the cell aggregate after the division. In addition, it is considered that the decrease in cell survival rate can be suppressed by increasing the frequency of backflow washing of the mesh structure (i.e., at time point when a predetermined number of cell aggregates have passed through a unit area of the mesh structure) since more cell aggregates pass through the mesh structure when the cell density is higher.

### [Experimental Example 7] Division of cell aggregate of human pluripotent stem cells (hiPS cells)

The hiPS cells were suspension cultured to form a cell aggregate, the cell aggregate was divided using the above-mentioned mesh structure, and a test was conducted to confirm the division performance and dividing process of the mesh structure by observing the survival rate of the cell aggregate after each division for each volume.

### (Three-dimensional culture of hiPS cell before division)

### Medium 1:

Liquid medium composition prepared by injecting 0.016% (w/v) deacylated gellan gum (KELCOGEL CG-LA, manufactured by Saneigen FFI) using FCeM-series Preparation Kit (manufactured by Nissan Chemical Corporation) to mTeSR1 medium (manufactured by STEMCELL Technologies) containing 10 µM Y-27632 (manufactured by FUJIFILM Wako Pure Chemical Corporation) according to the mixing method described in patent document 2.

### Medium 2:

Liquid medium composition prepared by injecting 0.016% (w/v) deacylated gellan gum using FCeM-series Preparation Kit to mTeSR1 medium according to the mixing method described in patent document 2.

The hiPS cell line 253G1 (distributed from RIKEN) was maintenance cultured in a CO₂ incubator (37°C, 5% CO₂) in a static state using a variable volume 200 mL culture bag (manufactured by Nipro), medium 1 and medium 2.

The cells were seeded in medium 1 on day 0 of culture, medium 2 was added every 1 to 3 days, and this was continued for 6 to 8 days to form a cell aggregate. On the final day, the cell aggregate was collected using MACS (registered trade mark) Smart Stratiners (70 µm, manufactured by MACS), suspended in medium 1, and then passed through a device according to the device of the present invention, and the divided cell aggregates were seeded (day 0). This was repeated to carry out maintenance culture of the cells.

### (Specifications of the mesh structure)

The instruments used in the following cases were sterilized with ethanol for disinfection.

As an example product of the mesh structure, a porous film having the following shape was produced.
(a) The shape of the opening is the regular hexagon shown in Fig. 3(a), and the cross-sectional shape of the beam part is the rectangle shown in Fig. 3(c).
(b) The shape of the opening is the square shown in Fig. 5(a), and the cross-sectional shape of the beam part is the rectangle shown in Fig. 3(c).
(c) The shape of the opening is the regular hexagon shown in Fig. 3(a), and the cross-sectional shape of the beam part is the shape (having a circular arc and a chord) with rounded corners on the inlet side shown in Fig. 3(e)).
(d) The shape of the opening is the square shown in Fig. 5(a), and the cross-sectional shape of the beam part is the shape (having a circular arc and a chord) with rounded corners on the inlet side shown in Fig. 3(e)).

The material of the film body is nickel, the thickness of the film body (thickness t1 in Figs. 3(c), (e)) is 20 µm, and the wire diameter (width of the beam part) is 50 µm. The pore size of each through-hole (the distance between two parallel sides facing each other among the six sides of the regular hexagon which is the shape of the opening or the distance between the two sides of a square, which is the shape of the opening) is 60 µm.

The shape of the outer circumference of the film body is circular, and the size (diameter) of the circle is 6 mm.

### (Holder for holding the device of the present invention)

As shown in Fig. 6, a holder for setting the mesh structure was produced. The inner diameter of the conduit (circular cross section) for flowing the liquid medium composition in which cell aggregates are dispersed is 3.0 mm, and the effective diameter of the flow of the liquid medium composition passing through the mesh structure is also 3.0 mm.

### (Division of cell aggregate of hiPS cell)

After suspension culture for 7 days, the cell aggregates were collected using MACS (registered trade mark) Smart Stratiners (70 µm, manufactured by MACS), suspended in medium 1 to a cell density of 3.0×10⁵ cells/mL. 45 mL of each suspension was transferred to a 50 mL syringe (manufactured by Nipro), the suspension was passed through an example product of the device of the present invention at a processing speed of 10 cm/sec, and dispensed into a 15 mL tube every 15 mL.

In addition, division including a backflow washing step was also performed by returning syringe with 1 mL every 10 mL.

The 15 mL tube after dispensing was allowed to stand in an incubator (37°C, 5% CO₂) .

### (Cell survival rate)

After 2 hours from the division, the dispensed 15 mL tube was removed from the incubator, the cell aggregates were well dispersed by blending with inversion. 0.75 mL of the culture medium was collected and 0.75 mL of ATP reagent (CellTiter-Glo (registered trade mark) Luminescent Cell Viability Assay, manufactured by Promega) was added and the mixture was well stirred with Pipetman. After allowing to stand at room temperature for 10 min, 100 µL of each was dispensed into a white 96-well plate, the luminescence intensity (RLU value) was measured with Enspire (manufactured by Perkin Elmer), and the number of viable cells was measured by subtracting the luminescence value of the medium alone.

The relative value when the RLU value (ATP measurement, luminescence intensity) of the suspension before division was 100% was taken as the cell survival rate.

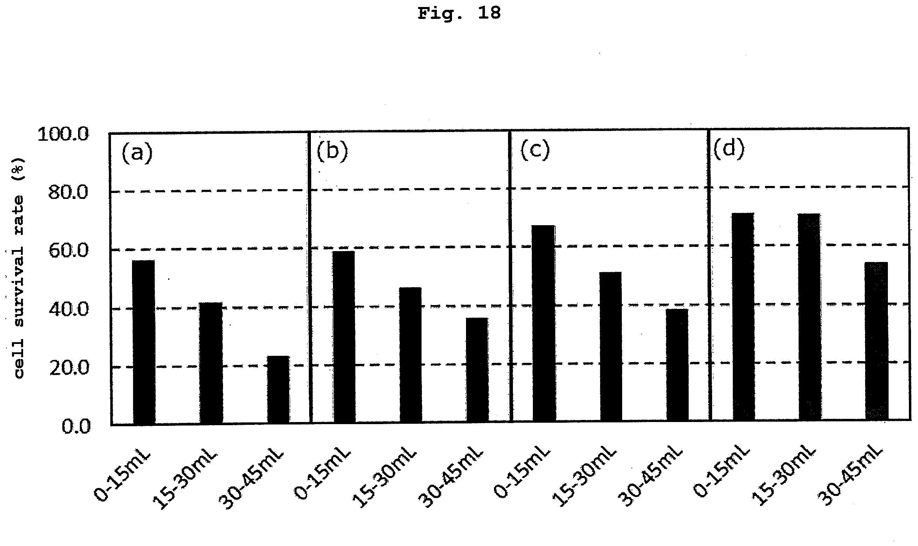

The results of the above are shown in the graph of Fig. 18.

As is clear from the results shown in the graph of Fig. 18, it was found that the cell survival rate decreases in a dose-dependent manner under all conditions. Furthermore, it was found that the cell survival rate is higher when the opening shape is square than when it is a regular hexagon, and that the cell survival rate is higher when the cross-sectional shape of the beam part is rounded at the corner on the inlet side than when it is rectangular.

The results regarding the shape of the opening in this test are not simply influenced by the shape of the opening. Since the opening area of the regular hexagon is 3118 µm² and the opening area of the square is 3600 µm², it is also considered that higher cell survival rate is achieved with the square having a larger opening area.

From the above, it was clarified that it is very effective, in the division when scaled up, to make the cross-sectional shape of the beam part a rectangular shape with rounded corners on the inlet side.

### [Experimental Example 8] Culture of human pluripotent stem cell (hiPS cell)

### (adhesion maintenance culture)

hiPS cell line 253G1 (distributed by RIKEN) cells were seeded in a 6 cm culture dish coated with vitronectin VTN-N (manufactured by ThermoFisher Scientific, #A14700) at 5.0×10⁴ cells - 5.0×10⁵ cells, and cultured using mTeSR (registered trade mark) 1 medium (manufactured by STEMCELL Technologies, #ST-05850). After seeding, the cells were cultured in mTeSR1 medium containing 10 µM Y-27632 (manufactured by FUJIFILM Wako Pure Chemical Industries, Ltd., #030-24021) for 24 hr, and thereafter the medium was replaced every day with mTeSR1 medium not containing 10 µM Y-27632. 3 - 4 days after seeding, the cells were detached using PBS solution containing 0.5 mM EDTA, whereby subculture was maintained.

### (Suspension maintenance culture)

Medium 1: Composition prepared using 0.016% (w/v) deacylated gellan gum (KELCOGEL CG-LA, manufactured by Saneigen FFI) and FCeM-series Preparation Kit (manufactured by Nissan Chemical Corporation) to mTeSR1 medium containing 10 µM Y-27632 according to the method of patent document 1 (WO 2016/163444)

Medium 2: Composition prepared using 0.016% (w/v) deacylated gellan gum and FCeM-series Preparation Kit to mTeSR1 medium according to the method of patent document 1

To carry out three-dimensional culture, the hiPS cell line 253G1 used for seeding was detached as a colony state using Dissociation Solution for human ES/iPS Cells (manufactured by REPROCELL, #RCHETP002).

After suspending the detached cells in medium 1, the cells were divided using a porous film (opening shape is regular hexagon, pore size (distance between two parallel sides) 70 µm, wire diameter (beam width) 20 µm, passage speed 10 cm/sec) to produce cell aggregates.

A part of the cells after division was separated and dissociated into single cells by TripLE Select (manufactured by ThermoFisher Scientific, #12563011) treatment, and then the number of viable cells was measured using a cell counting device (manufactured by ChemoMetec, #NC-200).

After adjusting the cell concentration to afford viable cells at 2.0×10⁵ cells/mL, the cells were seeded in a gas permeable culture bag (manufactured by Nipro, #87-352), and three-dimensionally cultured in a CO₂ incubator (37°C, 5% CO₂) in a stationary state.

On day 0 of culture, they were seeded in medium 1, medium 2 was added in 2 times the amount of the seeded amount on days 1 and 4 of culture, and the cells were cultured for 7 days. The formed cell aggregates were collected using a 70 µm mesh (manufactured by Miltenyi Biotec, #130-098-462), suspended in medium 1, and divided using a porous film (opening shape of regular hexagon, pore size (distance between parallel two sides) 70 µm, wire diameter (beam width) 20 µm, pass through speed 10 cm/sec), whereby subculture was maintained.

### [Experimental Example 9] Optimal cell seeding density in suspension culture

Using cells subcultured by the method described in Experimental Example 8, the optimal cell seeding density in suspension culture was verified.

Adhesion cultured hiPS cell line 253G1 cells were detached as a colony state using Dissociation Solution for human ES/iPS Cells (manufactured by REPROCELL, #RCHETP002). After suspending the detached cells in medium 1, the cells were divided using a porous film (opening shape is regular hexagon, pore size (distance between two parallel sides) 70 µm, wire diameter (beam width) 20 µm, passage speed 10 cm/sec) to produce cell aggregates.

A part of the cells after division was separated and dissociated into single cells by TripLE Select (manufactured by ThermoFisher Scientific, #12563011) treatment, and then the number of viable cells was measured using a cell counting device (manufactured by ChemoMetec, #NC-200).

After adjusting the cell concentration to afford viable cells at 1 or 2 or 4 or 6 or 8×10⁵ cells/mL, the cells were seeded in a 15 mL tube (manufactured by Falcon, model number 352095), and three-dimensionally cultured in a CO₂ incubator (37°C, 5%CO₂) in a stationary state without sealing with a lid.

On day 0 of culture, they were seeded in medium 1, medium 2 was added in 2 times the amount of the seeded amount on days 1 and 4 of culture, and the cells were cultured for 7 days.

### (Calculation of proliferation rate)

A part of the culture media under respective conditions was collected on days 0, 1, 2, 3, 4, 7 of culture, and ATP reagent (CellTiter-Glo (registered trade mark) Luminescent Cell Viability Assay, manufactured by Promega) in amounts equal to the culture medium was added to each and the mixture was stirred with Pipetman. After allowing to stand at room temperature for 10 min, 150 µL of each was dispensed into a white 96-well plate, the luminescence intensity (RLU value) was measured with Enspire (manufactured by Perkin Elmer), and the number of viable cells in the filtrate that passed through the mesh was measured by subtracting the luminescence value of the medium alone. The relative value on each measurement day with the RLU value (ATP measurement, luminescence intensity) of the culture medium on day 0 of culture as 100% was taken as the proliferation rate. The test results are shown in Table 8.

**[Table 8]**

| density of seeded cells (×10^5 cells/mL) | proliferation rate (%) | | | | | |
|---|---|---|---|---|---|---|
| | day 0 | day 1 | day 2 | day 3 | day 4 | day 7 |
| 1 | 100 | 58 | 84 | 147 | 219 | 288 |
| 2 | 100 | 109 | 140 | 206 | 260 | 485 |
| 4 | 100 | 124 | 190 | 228 | 259 | 351 |
| 6 | 100 | 120 | 182 | 183 | 200 | 259 |
| 8 | 100 | 111 | 167 | 138 | 135 | 202 |

As shown in Table 8, it was clarified that the proliferation rate on day 7 is the highest under conditions of cell seeding density being 2×10⁵ cells/mL. For efficient proliferation of cell requires seeding of cells at an appropriate concentration.

### [Experimental Example 10] Optimal medium addition method in suspension culture

Using cells subcultured by the suspension maintenance culture described in Experimental Example 8, optical medium addition method in suspension culture was verified.

hiPS cell line 253G1 cells on day 7 - 8 that was cultured for 1 passage or more by three-dimensional culture were collected, suspended in medium 1, and divided using a porous film (opening shape is regular hexagon, pore size (distance between two parallel sides) 70 µm, wire diameter (beam width) 20 µm, passage speed 10 cm/sec) to produce cell aggregates.

A part of the cells after division was separated and dissociated into single cells by TripLE Select (manufactured by ThermoFisher Scientific, #12563011) treatment, and then the number of viable cells was measured using a cell counting device (manufactured by ChemoMetec, #NC-200).

After adjusting the cell concentration to afford viable cells at 2×10⁵ cells/mL, the cells were seeded in a gas permeable culture bag (manufactured by Nipro, #87-352), and three-dimensionally cultured in a CO₂ incubator (37°C, 5%CO₂) in a stationary state. On day 1 of culture, 1 mL of the cell culture medium in the culture bag was seeded in a 15 mL tube (manufactured by Falcon, model number 352095), and medium 2 was added on days 1 - 6 of culture as described in Table 9. The 15 mL tube containing the seeded cells was three-dimensionally cultured without sealing with a lid in a CO₂ incubator (37°C, 5%CO₂) in a stationary state.

### (Calculation of proliferation rate)

On day 7 of culture, 1 mL of the culture medium under respective conditions was collected and 1 mL of ATP reagent (CellTiter-Glo (registered trade mark) Luminescent Cell Viability Assay, manufactured by Promega) was added and the mixture was stirred with Pipetman. After allowing to stand at room temperature for 10 min, 150 µL of each was dispensed into a white 96-well plate, the luminescence intensity (RLU value) was measured with Enspire (manufactured by Perkin Elmer), and the number of viable cells was measured by subtracting the luminescence value of the medium alone. The relative value on day 7 when the RLU value (ATP measurement, luminescence intensity) of the culture medium on day 1 of culture was 1 was taken as the proliferation rate. The test results are shown in Table 9.

### (Measurement of cell aggregate average diameter)

The cell aggregates were well dispersed. A part of the culture medium used in the test was placed in a 12 well plate, and the size of the cell aggregates with a diameter of 50 µm or more in the culture medium under each condition used in the test was measured. In the measurement of the size of the cell aggregates, the equivalent-circle-diameter of the cell aggregate was measured, and the mean thereof was taken as the average diameter.

**[Table 9]**

| condition | day 1 | day 2 | day 3 | day 4 | day 5 | day 6 | day 7 total amount of medium (mL) | proliferation rate (fold) | proliferation rate per amount of medium (fold/mL) | sphere average diameter (µm) |
|---|---|---|---|---|---|---|---|---|---|---|
| 1 | 0 | 0 | 0 | 0 | 0 | 0 | 1 | 0.5 | 0.5 | 109.2 |
| 2 | 1 | 0 | 0 | 0 | 0 | 0 | 2 | 2.0 | 1.0 | 134.9 |
| 3 | 1 | 0 | 1 | 0 | 0 | 0 | 3 | 2.9 | 1.0 | 146.4 |
| 4 | 1 | 0 | 0 | 1 | 0 | 0 | 3 | 3.9 | 1.3 | 140.7 |
| 5 | 1 | 0 | 0 | 0 | 0 | 1 | 3 | 4.0 | 1.3 | 137.5 |
| 6 | 2 | 0 | 0 | 0 | 0 | 0 | 3 | 3.1 | 1.0 | 136.8 |
| 7 | 2 | 0 | 2 | 0 | 0 | 0 | 5 | 4.8 | 1.0 | 151.5 |
| 8 | 2 | 0 | 0 | 2 | 0 | 0 | 5 | 5.9 | 1.2 | 154.4 |
| 9 | 2 | 0 | 0 | 3 | 0 | 0 | 6 | 7.4 | 1.2 | 163.3 |
| 10 | 2 | 0 | 0 | 4 | 0 | 0 | 7 | 8.0 | 1.1 | 172.9 |
| 11 | 2 | 0 | 0 | 0 | 0 | 2 | 5 | 6.3 | 1.3 | 137.5 |
| 12 | 3 | 0 | 0 | 0 | 0 | 0 | 4 | 4.1 | 1.0 | 148.6 |
| 13 | 3 | 0 | 3 | 0 | 0 | 0 | 7 | 7.2 | 1.0 | 167.0 |
| 14 | 3 | 0 | 0 | 2 | 0 | 0 | 6 | 6.6 | 1.1 | 167.7 |
| 15 | 3 | 0 | 0 | 3 | 0 | 0 | 7 | 7.6 | 1.1 | 168.1 |
| 16 | 3 | 0 | 0 | 4 | 0 | 0 | 8 | 9.6 | 1.2 | 172.7 |
| 17 | 3 | 0 | 0 | 0 | 0 | 3 | 7 | 7.2 | 1.0 | 162.3 |
| 18 | 4 | 0 | 0 | 0 | 0 | 0 | 5 | 4.7 | 0.9 | 146.0 |
| 19 | 4 | 0 | 4 | 0 | 0 | 0 | 9 | 8.9 | 1.0 | 188.9 |
| 20 | 4 | 0 | 0 | 2 | 0 | 0 | 7 | 8.4 | 1.2 | 167.5 |
| 21 | 4 | 0 | 0 | 3 | 0 | 0 | 8 | 9.7 | 1.2 | 182.2 |
| 22 | 4 | 0 | 0 | 4 | 0 | 0 | 9 | 10.3 | 1.1 | 179.7 |
| 23 | 4 | 0 | 0 | 0 | 0 | 4 | 9 | 8.4 | 0.9 | 178.3 |

As shown in Table 9, it was clarified that a higher amount of the medium added tends to lead to a higher proliferation rate and larger sphere average diameter. Efficient proliferation of cells requires addition of an appropriate amount of a medium at appropriate timing.

### [Experimental Example 11] Culture using gas permeable culture bag

Using cells subcultured by the suspension maintenance culture described in Experimental Example 8, suspension culture using gas permeable culture bag was verified.

hiPS cell line 253G1 cells on day 7 that were cultured for 1 passage or more by three-dimensional culture were collected, suspended in medium 1, and divided using a porous film (opening shape is regular hexagon, pore size (distance between two parallel sides) 70 µm, wire diameter (beam width) 20 µm, passage speed 10 cm/sec) to produce cell aggregates.

A part of the cells after division was separated and dissociated into single cells by TripLE Select (manufactured by ThermoFisher Scientific, #12563011) treatment, and then the number of viable cells was measured using a cell counting device (manufactured by ChemoMetec, #NC-200).

After adjusting the cell concentration to afford viable cells at 2.0×10⁵ cells/mL, the cells were seeded in a 200 mL gas permeable culture bag (manufactured by Nipro, #87-352) or 1 L gas permeable culture bag (manufactured by Nipro, #87-302), and three-dimensionally cultured in a CO₂ incubator (37°C, 5%CO₂) in a stationary state.

On day 0 of culture, they were seeded in medium 1, medium 2 was added in 2 times the amount of the seeded amount on days 1 and 4 of culture, and the cells were cultured for 7 days. After culture, the cells were collected using a mesh, suspended in medium 1, and divided using a porous film (opening shape of regular hexagon, pore size (distance between parallel two sides) 70 µm, wire diameter (beam width) 20 µm, pass through speed 10 cm/sec), whereby subculture was maintained.

### (Calculation of proliferation rate)

A part of the cells on day 7 of culture was separated and dissociated into single cells by TripLE Select (manufactured by ThermoFisher Scientific, #12563011) treatment, and then the number of viable cells was measured using a cell counting device (manufactured by ChemoMetec, #NC-200). The proliferation rate was calculated by dividing the number of cells on day 7 of culture by the number of cells seeded on day 0 of culture.

### (Measurement of number and average diameter of cell aggregates)

The cell aggregates were well dispersed. A part of the culture medium used in the test was placed in a 12 well plate, and the number and size of the cell aggregates with a diameter of 50 µm or more in the culture medium was measured. In the measurement of the size of the cell aggregates, the equivalent-circle-diameter of the cell aggregate was measured, and the mean thereof was taken as the average diameter.

**[Table 10]**

| culture bag scale | 200 mL | 1000 mL |
|---|---|---|
| number of seeded cells (×10^6 cells) | 8 | 40 |
| number of cells 7 days later (×10^6 cells) | 80.4 | 355 |
| proliferation rate (fold) | 10.1 | 8.9 |
| sphere average diameter 7 days later (µm) | 170.2 | 153.1 |
| total number of spheres 7 days later (spheres) | 62400 | 330000 |

As shown in Table 10, the proliferation rate when the 200 mL culture bag was used was about 10-fold, and the proliferation rate when the 1 L culture bag was used was about 9-fold. From the above results, it was clarified that the culture can be performed with almost the same efficiency even on the 200 mL scale and the 1 L scale.

### [Experimental Example 12] Culture using various containers

Using cells subcultured by the suspension maintenance culture described in Experimental Example 8, suspension culture using various containers was verified.

hiPS cell line 253G1 cells on day 7 - 8 that were cultured for 1 passage or more by three-dimensional culture were collected, suspended in medium 1, and divided using a porous film (opening shape is regular hexagon, pore size (distance between two parallel sides) 70 µm, wire diameter (beam width) 20 µm, passage speed 10 cm/sec) to produce cell aggregates.

A part of the cells after division was separated and dissociated into single cells by TripLE Select (manufactured by ThermoFisher Scientific, #12563011) treatment, and then the number of viable cells was measured using a cell counting device (manufactured by ChemoMetec, #NC-200).

After adjusting the cell concentration to afford viable cells at 2.0×10⁵ cells/mL, the cells were seeded in a 50 mL tube (manufactured by WATOSON, #1342-050S), low adhesive 10 cm dish (manufactured by Corning, #3262), cell culture flask (manufactured by Corning, #430168), and three-dimensionally cultured in a CO₂ incubator (37°C, 5%CO₂) in a stationary state.

At that time, the 50 mL tube and the cell culture flask were cultured without sealing with a lid. On day 0 of culture, they were seeded in medium 1, medium 2 was added in 2 times the amount of the seeded amount on days 1 and 4 of culture, and the cells were cultured for 7 days. The culture conditions and the results are shown in Table 11. Under condition 2, the operation of suspending the culture medium was performed on days other than the day when the medium was added.

### (Calculation of proliferation rate)

A part of the cells on day 7 of culture was separated and dissociated into single cells by TripLE Select (manufactured by ThermoFisher Scientific, #12563011) treatment, and then the number of viable cells was measured using a cell counting device (manufactured by ChemoMetec, #NC-200). The proliferation rate was calculated by dividing the number of cells on day 7 of culture by the number of cells seeded on day 0 of culture.

### (Measurement of number and average diameter of cell aggregates)

The cell aggregates were well dispersed. A part of the culture medium used in the test was placed in a 12 well plate, and the number and size of the cell aggregates with a diameter of 50 µm or more in the culture medium was measured. In the measurement of the size of the cell aggregates, the equivalent-circle-diameter of the cell aggregate was measured, and the mean thereof was taken as the average diameter.

**[Table 11]**

| condition | culture container | proliferation rate | sphere average diameter (µm) | number of spheres in 50 mL of culture medium (spheres) | bottom area (cm^2) | air contact area (cm^2) | liquid surface level (cm) |
|---|---|---|---|---|---|---|---|
| 1 | 50 mL tube | 3.6 | 125.4 | 16100 | 6.2 | 6.2 | 10.3 |
| 2 | 50 mL tube | 4.0 | 129.5 | 13700 | 6.2 | 6.2 | 10.3 |
| 3 | low adhesive 10 cm dish | 11.0 | 148.0 | 24950 | 60.8 | 60.8 | 0.9 |
| 4 | cell culture flask | 4.6 | 132.0 | 19400 | 10.5 | 10.5 | 4.6 |

As shown in Table 11, it was clarified that a proliferation rate of the same level as that of the gas permeable culture bag shown in Table 10 can be obtained by using a low adhesive 10 cm dish as the culture container. In addition, the area where the culture medium in each container on day 7 of culture was in contact with air (indicated as air contact area) and the height of the culture medium (indicated as liquid surface level) were measured. It is considered that the reason for the improved proliferation rate in the low adhesive 10 cm dish as compared with other containers is that the area of the culture medium in contact with air is large and the level of the culture medium is low. From the above results, it was suggested that efficient proliferation of cells requires exchange of gases necessary for the growth of cells in the medium.

### [Experimental Example 13] Collection of cell aggregate of human pluripotent stem cell (hiPS cell)

Using cells subcultured by the suspension maintenance culture described in Experimental Example 8, the collection conditions of the cell aggregate were verified. A cell culture medium of hiPS cell line 253G1 on day 7 that was cultured for 1 passage or more by three-dimensional culture was passed through a mesh container for collection under various conditions different in the effective area and shape at a speed of 1 mL/sec to trap the cell aggregates in the mesh. Thereafter, the cell aggregates were collected by backflow of the fresh medium 2 described in Experimental Example 8.

### (Specifications of collector)

Under conditions 1 and 2, a collector container in which the space in the collection container is divided into two by a mesh as shown in Fig. 9 was used.

In the example shown in Fig. 9(a), the outer circumference shape of the inner space of the collection container is a circular shape. Under condition 1, a collector container in which the outer circumference shape of the inner space of the collection container is rectangle (long side is present in the direction from the inlet to the outlet), a mesh pore size is 65 µm and the effective area is about 13 cm² was used. Under condition 2, a collector container in which the outer circumference shape (basic shape) of the inner space of the collection container is a rhombus (inlet and outlet are provided at two opposing apex parts), a mesh pore size is 65 µm and the effective area is about 34 cm² was used. The trapped cell aggregates were moved to the collection container by reversing the flow direction at the time of collection with respect to the flow direction at the time of trapping.

### (Measurement of number and average diameter of cell aggregates)

The cell aggregates were well dispersed. A part of the culture medium used in the test was placed in a 12 well plate, and the number and size of the cell aggregates with a diameter of 50 µm or more in the culture medium was measured. In the measurement of the size of the cell aggregates, the equivalent-circle-diameter of the cell aggregate was measured, and the mean thereof was taken as the average diameter.

### (Calculation of collection rate)

The culture medium, and 1 mL of the collection solution were collected, 1 mL of ATP reagent (CellTiter-Glo (registered trade mark) Luminescent Cell Viability Assay, manufactured by Promega) was added to each and the mixture was stirred with Pipetman. After allowing to stand at room temperature for 10 min, 150 µL of each was dispensed into a white 96-well plate, the luminescence intensity (RLU value) was measured with Enspire (manufactured by Perkin Elmer), and the number of viable cells in the filtrate that passed through the mesh was measured by subtracting the luminescence value of the medium alone. The relative value of the collection solution with the RLU value (ATP measurement, luminescence intensity) of the culture medium as 100% was taken as the collection rate.

The results of the collection rate are shown in Table 12.

**[Table 12]**

| | condition 1 | condition 2 |
|---|---|---|
| shape of collector | Fig. 9 | Fig. 9 |
| effective area (cm2) | 13 | 34 |
| mesh pore size (µm) | 65 | 65 |
| speed (mL/sec) | 1 | 1 |
| passage speed (cm/sec) | 0.10 | 0.03 |
| number of cell aggregates (cell aggregates/mL) | 199 | 321 |
| total number of cell aggregates (cell aggregates) | 9950 | 64200 |
| number of cell aggregates per unit area (cell aggregates/cm2) | 765 | 1888 |
| cell aggregate average diameter (µm) | 151.1 | 175.7 |
| collection rate (%) | 79.3 | 82.4 |

As shown in Table 12, a collection rate of around 80% was obtained under conditions 1 and 2. The collection rate was improved under conditions 1 and 2 in Table 12 as compared with conditions 9 and 10 in Experimental Example 5. From these results, it was clarified that the collection efficiency can be improved by optimizing the shape of the collector and the mesh pore size, and the cell aggregate can be collected efficiently.

### [Experimental Example 13] Closed system culture

Using cells subcultured by the suspension maintenance culture described in Experimental Example 8, the closed system culture shown in Fig. 7 was verified.

hiPS cell line 253G1 cells on day 7 of culture that were cultured for 1 passage or more by three-dimensional culture were collected, suspended in medium 1, and divided using a porous film (opening shape is regular hexagon, pore size (distance between two parallel sides) 70 µm, wire diameter (beam width) 20 µm, passage speed 10 cm/sec) to produce cell aggregates.

A part of the cells after division was separated and dissociated into single cells by TripLE Select (manufactured by ThermoFisher Scientific, #12563011) treatment, and then the number of viable cells was measured using a cell counting device (manufactured by ChemoMetec, #NC-200).

After adjusting the cell concentration to afford viable cells at 2.0×10⁵ cells/mL, the cells were seeded in a gas permeable culture bag (manufactured by Nipro, #87-352), and three-dimensionally cultured in a CO₂ incubator (37°C, 5%CO₂) in a stationary state. On day 0 of culture, they were seeded in medium 1, medium 2 was added in 2 times the amount of the seeded amount on days 1 and 4 of culture, and the cells were cultured for 7 days.

On day 7 of culture, the cell aggregates were collected by connecting to a closed system flow path having the collector and divider shown in Fig. 7 and a new culture bag, and the grown cell aggregates were divided and seeded into the new culture bag, whereby maintenance culture was performed. The concentration was adjusted to afford viable cells at 2×10⁵ cells/mL.

As the collector, a separation mesh having pore size 65 µm, wire diameter 48 µm, effective area about 34 cm² was used, and a porous film (opening shape is regular hexagon, pore size (distance between two parallel sides) 70 µm, wire diameter (beam width) 20 µm, passage speed 10 cm/sec) was used as the divider.

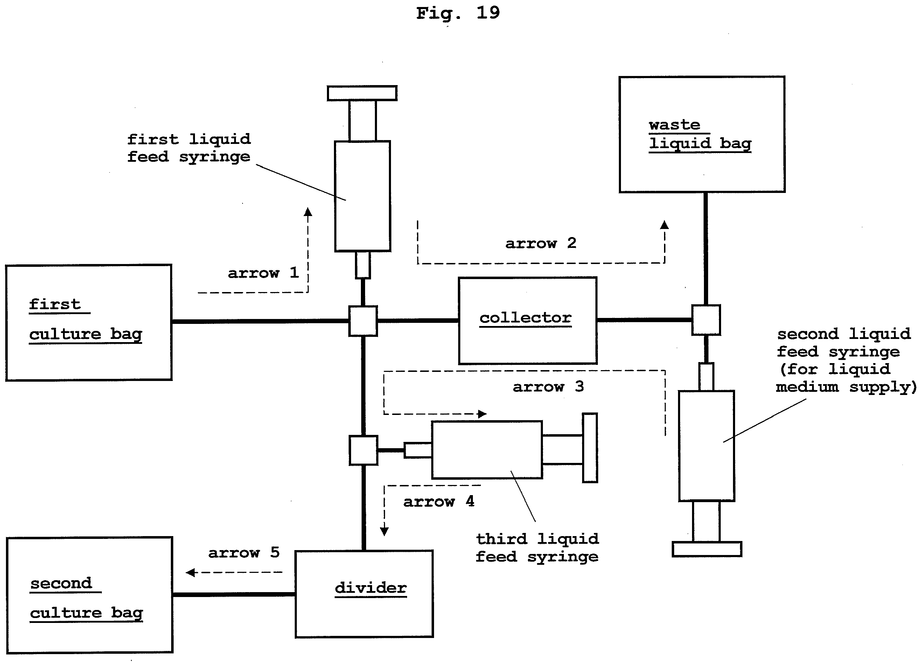

The constitution of the cell culture system actually used in this Experimental Example is shown as a block diagram in Fig. 19. The small squares in the figure are switching valves that switch the flow path so that the suspension or liquid medium will flow along the dashed arrows in the figure. Accessory constitutions such as elements and piping for appropriately adding a liquid medium from the outside for the purpose of adjusting the concentration of cell aggregates, elements and piping for extracting a suspension for inspection of cell aggregates, and the like are not shown.

As shown in Fig. 19, by the operation of the first liquid feed syringe, the suspension (liquid medium containing cell aggregates) in the first culture bag enters the first liquid feed syringe along arrow 1, and then enters the collector along arrow 2. Here, only the cell aggregate remains, and the liquid medium used for culture passes through the collector and enters the waste liquid bag. Here, the collector is located prior to the divider and is used to replace the liquid medium before dividing. Next, a fresh liquid medium is fed out from the second feed syringe along arrow 3 and enters the third liquid feed syringe as a new suspension with the cell aggregates in the collector. The new suspension is then fed to the divider along arrow 4, where the cell aggregate is divided and enters the second culture bag along arrow 5.

In the example of Fig. 19, the first culture bag is the supply source in Fig. 1 and the second culture bag is the first container in Fig. 1. The second culture bag can be replaced with the first culture bag in a closed manner by switching the conduit, and the like. The cell aggregate in the second culture bag can be divided again by the movement along the aforementioned arrows 1-5 and can enter the third culture bag (not shown).

### (Calculation of proliferation rate)

A part of the cells on days 0 and 7 of culture was separated and dissociated into single cells by TripLE Select (manufactured by ThermoFisher Scientific, #12563011) treatment, and then the number of viable cells was measured using a cell counting device (manufactured by ChemoMetec, #NC-200). The proliferation rate was calculated by dividing the number of cells on day 7 of culture by the number of cells seeded on day 0 of culture. The measurement results are shown in Table 13.

### (Calculation of collection rate)

The culture medium, filtrate, and 1 mL of the collection solution were collected, 1 mL of ATP reagent (CellTiter-Glo (registered trade mark) Luminescent Cell Viability Assay, manufactured by Promega) was added to each and the mixture was stirred with Pipetman. After allowing to stand at room temperature for 10 min, 150 µL of each was dispensed into a white 96-well plate, the luminescence intensity (RLU value) was measured with Enspire (manufactured by Perkin Elmer), and the number of viable cells in the filtrate that passed through the mesh was measured by subtracting the luminescence value of the medium alone. When the RLU value (ATP measurement, luminescence intensity) of the culture medium was 100%, the relative value of the filtrate was taken as the filtrate outflow cells and the relative value of the collection solution was taken as the collection rate. The measurement results are shown in Table 13.

### (Measurement of number and average diameter of cell aggregates)

The cell aggregates were well dispersed. A part of the culture medium used in the test was placed in a 12 well plate, and the size of the cell aggregates with a diameter of 50 µm or more in the culture medium was measured. In the measurement of the size of the cell aggregates, the equivalent-circle-diameter of the cell aggregate was measured, and the mean thereof was taken as the average diameter. The measurement results are shown in Table 13.

### (Evaluation of undifferentiated state)

A part of the cells on day 7 of culture was separated and dissociated into single cells by TripLE Select (manufactured by ThermoFisher Scientific, #12563011) treatment, suspended in 4% PFA solution, and immobilized by allowing to stand for 15 min.

The immobilized cells were stained by suspending in anti-TRA-1-60 antibody (BD, #560193) and anti-SSEA-4 antibody (BD, #560796) solution and allowing to stand for 30 min. The cells were washed with PBS solution containing 2% FBS, and the expression of TRA-1-60 and SSEA-4, which are indicators of the undifferentiated state of iPS cells, was measured using Fortessa X-20(BD). The measurement results are shown in Table 14.

**[Table 13]**

| | |
|---|---|
| proliferation rate (fold) | 9.4 |
| collection rate (%) | 72.9 |
| cells that passed mesh (%) | 8.8 |
| cell aggregate average diameter (µm) | 162.1 |

**[Table 14]**

| | proportion of TRA-60 positive, SSEA-4 positive cell population (%) |
|---|---|
| after 3 passages | 77.3 |

As shown in Tables 13 and 14, it was clarified that undifferentiated culture can be performed while maintaining a good proliferation rate in the cell culture system (that is, cell culture system using a closed system flow path).

### [Industrial Applicability]

According to the present invention, it is possible to perform division of cell aggregates and further suspension culture of the divided cells in a closed system, and further repeat division and culture of cell aggregates while maintaining the closed system, and preferably perform proliferation of cell aggregates. In addition, it is possible to collect the proliferated cell aggregates while maintaining the closed system.

This application is based on a patent application No. 2018-148042 filed in Japan (filing date: August 6, 2018) and a patent application No. 2018-170103 filed in Japan (filing date: September 11, 2018), the contents of which are incorporated in full herein.

### [Explanation of Symbols]

- 10: first container
- 20: divider
- 22: mesh structure
- S1: supply source
- P1: conduit
- P2: conduit
- P3: conduit
- F1: liquid feeding device

## Claims

1. A cell culture system for dividing and subculturing a cell aggregate, comprising at least:
a divider for dividing a cell aggregate to be divided which is supplied together with a liquid medium from a supply source into smaller cell aggregates; and
a first container for suspension culturing the cell aggregates divided by the divider;
wherein,
the divider has an inlet, an internal dividing conduit, and an outlet, the inlet is constituted such that the cell aggregate to be divided and the liquid medium are received from the supply source into the dividing conduit, the dividing conduit is provided with a mesh structure to divide the cell aggregate to be divided, the cell aggregate to be divided is divided when passing through the mesh structure together with the liquid medium, and the outlet is connected to the first container so as to deliver the divided cell aggregates to the first container, and
the first container has a constitution for receiving the divided cell aggregates and the liquid medium and sending out the cell aggregates suspension cultured in the first container.

2. The cell culture system according to claim 1, wherein the first container is a flexible cell culture bag.

3. The cell culture system according to claim 1 or 2, wherein the mesh structure is a mesh woven with wire.

4. The cell culture system according to claim 1 or 2, wherein
the mesh structure is a porous film with many through-holes disposed on the film surface, the mesh structure comprises many through-holes penetrating the predetermined region in the film thickness direction, and a beam part serving as a partition between the through-holes,
the through-holes have an opening shape of a size permitting passage of the smaller cell aggregates,
and the beam part is a remainder after subtracting the through-hole from the main body part in the predetermined region, is a part that cuts the cell aggregates to be divided, and is integrally connected to form a network.

5. The cell culture system according to claim 4, wherein a cross-sectional shape in the perpendicular longitudinal direction of the beam part is a rectangle, or two corners on the inlet side of the rectangle have a round shape.

6. The cell culture system according to claim 4 or 5, wherein
said many through-holes have opening shapes of quadrangles congruent with each other, and said beam parts are connected to each other in an orthogonal lattice pattern, or said many through-holes have opening shapes of hexagons congruent with each other, and said beam parts are connected to each other in a honeycomb-shape.

7. The cell culture system according to any one of claims 1 to 6, further comprising a collector for recovering cell aggregates with a predetermined size, wherein
the collector is connected to the first container so as to receive the cell aggregates suspension cultured in the first container and the liquid medium,
the collector has a separation chamber, the separation chamber is divided into a first chamber and a second chamber by a mesh structure for separation, and an internal flow path is configured such that the liquid medium that has entered the collector moves from the first chamber through the mesh structure for separation to the second chamber and exits the collector, and
the mesh structure for separation has mesh-holes of a predetermined size to prevent cell aggregates of a size intended for collection from passing through, whereby, of the cell aggregates that entered the collector together with the liquid medium, a cell aggregate of a size intended for collection does not pass through the mesh structure for separation and stays in the first chamber.

8. The cell culture system according to claim 7, wherein an overall shape of the mesh structure for separation is sheet-like or sac-like.

9. The cell culture system according to claim 7 or 8, wherein the mesh structure for separation is configured in a separation chamber of the collector such that the liquid medium has a vertically upward directional component in the progress direction when the liquid medium passes through mesh-holes of the mesh structure for separation in the collector.

10. The cell culture system according to any one of claims 7 to 9, wherein
the first chamber in the separation chamber of the collector comprises
a first inlet port for receiving the cell aggregates suspension cultured in the first container and the liquid medium in the first chamber,
a second inlet port for flowing a liquid for diluting a reagent and/or the suspension containing the cell aggregates and the liquid medium into the first chamber from the outside, and
an outlet port for flowing a part of the cell aggregates from the first chamber to the outside.

11. The cell culture system according to any one of claims 1 to 10, further comprising a liquid feeding device for moving the liquid medium and the cell aggregates, wherein the liquid feeding device is one or more selected from the group consisting of an apparatus for deforming the first container by compression to send out the liquid medium and the cell aggregates contained therein, a syringe pump, and a peristaltic pump.

12. The cell culture system according to any one of claims 1 to 11, further comprising
a backward direction liquid feeding function or a backward direction liquid feeding device for moving the predetermined liquid such that the predetermined liquid passes through the mesh structure in a direction opposite to the direction of passage of the cell aggregate to be divided through the mesh structure together with the liquid medium.

13. The cell culture system according to any one of claims 1 to 12, wherein the cell aggregate to be divided is a cell aggregate composed of pluripotent stem cells.

14. The cell culture system according to any one of claims 1 to 13, wherein the liquid medium comprises a fine structure for suspending the cells or cell aggregates in the liquid medium, and the fine structure is dispersed and floating in the liquid medium.

15. A method for producing a cell aggregate by using the cell culture system according to any one of claims 1 to 14, comprising
a step of dividing a cell aggregate to be divided and a liquid medium by passing the cell aggregate through the divider in the cell culture system, and
a step of feeding the cell aggregate divided in the divider into the first container together with a liquid medium, and performing suspension culture in the first container.

16. The production method according to claim 15, wherein the flow velocity of the liquid medium is 10 mm/sec - 500 mm/sec when the cell aggregate passes through the divider together with the liquid medium.

17. The method according to claim 15 or 16, further comprising a backflow washing step, wherein the backflow washing step is a step of passing, after division of a predetermined amount of the cell aggregates in the step of dividing the cell aggregates, a predetermined liquid through the mesh structure in the divider direction opposite to the direction of passage of the cell aggregate through the mesh structure of the device for division, thereby washing the mesh structure.

18. The production method according to any one of claims 15 to 17, wherein
the cell culture system is the cell culture system according to any one of claims 7 to 10,
the production method further comprising a step of feeding the cell aggregates suspension cultured in the first container into the collector of the cell culture system together with the liquid medium, and feeding and collecting cell aggregates remaining in the first chamber of the collector in a collection container.

19. The production method according to claim 17, wherein a flow velocity of the liquid medium passing through the collector for separation of the cell aggregates from the liquid medium is 0.01 mm/sec - 25 mm/sec.
